# EUROPEAN PATENT APPLICATION

(11) **EP 4 752 894 A1**
(43) Date of publication of application: **03.06.2026**
(21) Application number: 25155084.4
(22) Date of filing: 30.01.2025
(51) Int. Cl.: G16H 20/10, G16H 50/20, G16H 50/70, A61P 3/04, G06N 20/00

(54) **MACHINE LEARNING ARCHITECTURES TO DETERMINE USER RESPONSIVENESS BASED ON MULTI-MODAL MEASUREMENT DATA**

(30) Priority: 27.11.2024 US 202418962355
(71) Applicant: Click Therapeutics, Inc., New York, NY 10013 (US)
(72) Inventor: ADLER, Samantha, New York, 10013 (US); DIAZ CORTES, Margarita, New York, 10013 (US)
(74) Representative: Marks & Clerk LLP

(57) **Abstract**

Provided herein are systems and methods for selecting a weight loss medication using a machine learning model to address obesity. A computing system may receive physiological measurements of a user. The computing system may apply the measurements to a machine learning model. The machine learning model may be trained using a plurality of examples, each example comprising sample physiological measurements of a sample user and a corresponding sample weight loss medication administered to the sample user. The computing system may generate based on applying the one or more physiological measurements to the machine learning model, a metric indicating an expected outcome associated with a weight loss medication for the user. The computing system may provide to a user device associated with the user, a message indicating the expected outcome associated with the weight loss medication. The computing system can improve efficacy of the medication in addressing the obesity.

## Description

### CROSS-REFERENCE TO RELATED APPLICATIONS

The present application claims priority to and the benefit of U.S. Patent Application No. 18/962,355, titled "MACHINE LEARNING ARCHITECTURES TO DETERMINE USER RESPONSIVENESS BASED ON MULTI-MODAL MEASUREMENT DATA", and filed on November 27, 2024, the entire contents of which are hereby incorporated by reference in entirety.

### BACKGROUND

Obesity refers to an excess fat accumulation which is associated with increased body weight and health risks. This condition may arise from a diverse array of underlying factors, such as lifestyle factors, genetics, environmental factors, or medical conditions. For example, specific underlying factors that may lead to obesity include lifestyle choices (e.g., poor diet, overeating, or physical inactivity), genetics (e.g., inherited traits), psychological conditions (e.g., depression, stress, anxiety, or binge eating), medical conditions (e.g., hyperthyroidism), certain medications (e.g., steroids, or antidepressants), or hormones (e.g., leptin resistance), among others. At the molecular level, for individuals with obesity, fat cells (known as adipocytes) increase in size or number, causing the fat cells to store excess energy in the form of fat. Furthermore, irregular hormone activity, such as leptin resistance where the brain fails to respond effectively to signals regarding energy stores, may cause individuals to overeat despite high energy stores.

Obesity negatively impacts the mental health, physical health, and social well-being of those individuals that are affected. Individuals with obesity are at a heightened risk for a variety of physical health issues, such as cardiovascular diseases (e.g., hypertension, or heart failure), cardiometabolic conditions (e.g., diabetes), respiratory problems (e.g., sleep apnea, or asthma), or cancer (e.g., kidney, liver, or pancreatic), among others. There is also a plethora of mental health issues that individuals with obesity may face, such as depression, anxiety, social isolation, or chronic stress, among others. The daily quality of life of individuals with obesity is not only affected by health issues, but also accessibility (e.g., mobility impairment) and economic burden (e.g., medical expenses).

Certain obesity medications can be administered to individuals in an attempt to facilitate weight loss. Even with these drugs, however, there are side effects making it difficult for users to adhere to their individual prescription and subsequently dropping out of taking the medication. Furthermore, failing to take into account an individual's physiological measurements and other relevant information can lead to prescriptions resulting in a plurality of side effects, thereby causing the user to stop taking the medication and ultimately little to no improvement in health outcomes. In addition, certain individuals may be physiologically receptive to certain types of obesity medications, over others. Administering the same ineffective medication to such individuals can also result in little to no improvements in clinical outcomes. Adherence to the medication is a key determinant of success and reducing weight. However, when the prescription leads to multiple side effects with ranging degrees of severity, individuals may struggle to adhere to their prescription and achieve weight loss goals.

One approach to address medication adherence includes a trial-and-error method. Under this method, an individual is prescribed with a weight loss medication and then subsequently monitoring the individual's reaction and side effects to the prescription. There are, however, several drawbacks with the trial-and-error approach. For one, the method involves starting the individual on one medication, waiting to gather additional data to determine whether the medication is effective, and then switching to another medication if it is determined that the originally prescribed drug is ineffective or has side effects. This can lead to prolonged periods where the individual remains untreated or experiences suboptimal clinical outcomes. For another, each new drug introduces the potential for side effects or adverse events to the individual. Monitoring the individual for side effects or other events adds complexities, entailing frequent check-ins and collection of data from the individual. In addition, weight loss medications also present unique challenges. Weight loss response to medications varies widely among individuals due to differences in genetics, metabolism, baseline body composition, and underlying health conditions. The wide variance in individual responsiveness can result in certain individuals not adhering to the medication.

### SUMMARY

To address these and other technical problems with adherence to obesity medications, a digital therapeutic application can use a machine learning model as detailed herein that leverages real-time, multi-modal data from an individual to output expected outcomes to various weight loss medications, such as side effects, weight loss, and dropout rate, among others. There are several advantages for the users of the digital therapeutic application detailed herein. For one, the digital therapeutic application can aggregate and apply data in real-time in a wide variety of modalities from various sources to the machine learning model to personalize selection of weight loss medication. By using the machine learning model to analyze data from the user, the digital therapy application can identify which obesity medication is likely to yield the highest weight loss outcomes with the fewest side effects for that particular user. The output can also include precise medication type, dosage, frequency, and administration regimens for the weight loss medication. Relative to the trial-and-error approach, the machine learning model provides outputs for the most effective treatment and optimal clinical outcome for a specific user.

For another, the digital therapeutic application may use the machine learning model to forecast potential side effects based on the user data. The prediction on side effects is valuable for weight loss drugs that are known to have a wide range of possible adverse effects. By identifying these risks of side effects in advance, the digital therapeutic application can provide for adjustments to the administration of the obesity drug (e.g., type of drug, dosage, and frequency) to mitigate side effects before they arise. This proactive approach minimizes user discomfort, enhances adherence, and ultimately contributes to more successful outcomes. In addition, the machine learning model can be leveraged to identify a particular user at risk of dropping off from the weight loss medication. Using the model, the digital therapeutic application identifies behavioral and physiological factors that typically lead to discontinuation for that type of user and can provide different medication or administration parameters to avoid discontinuation. By offering personalized support, the digital therapeutic application can provide specific interventions to mitigate both side effects and dropout risk of the user, thereby increasing the likelihood of medication adherence and thus better clinical outcomes.

In addition, instances of the machine learning model are created and adapted for particular users to factor in the unique characteristics of each user. For instance, multiple instances of a machine learning model could be created for different individuals overseen by a physician, with each instance tailored to each individual's specific response profiles and preferences. For an individual with previous experience with side effects to a pre-disposition to a particular medication, an instance of the machine learning model for this individual is created to output higher probability of side effects for that medication. For another individual with no concerns with the same medication but difficulties with drug adherence due to their work schedule, another instance of the machine learning model is trained to account for factors known to lead to low adherence (e.g., higher frequency of administration). Since each instance of the machine learning model has been personalized for these individuals, these have the ability to generate different outputs even with same or similar measurement data. This enhances the precision and relevance of the outputs of the machine learning model across diverse user profiles and characteristics.

Moreover, the digital therapeutic application incorporates real-time data on the user's physiological measurements (e.g., blood glucose, heart rate, or blood pressure) from the user, to iteratively update risk of side effects and weight loss predictions while the user is adhering to the medication prescription. By continuously monitoring the user's measurements in real-time as the user progresses through their weight loss journey, the digital therapeutic application is able to iteratively continuously update the user's medication, dosage, and administration parameters, as well as the user's drop-out and side-effect risks. Unlike other approaches that do not incorporate real-world data, the digital therapeutic application assesses real-time data using the machine learning model to consider all relevant factors that may influence a user's response to specific weight loss medications. The use of the digital therapeutic application described herein enables the creation of highly personalized prescriptions while optimizing the integration of pharmacological and digital therapeutic interventions.

From a computer perspective, the machine learning model is capable of processing and analyzing large datasets across various modalities with high efficiency. By using the machine learning model, the digital therapeutics application can distill complex, high-dimensional data into an embedded representation, reducing computation time without compromising predictive accuracy. Compared to trial-and-error approaches, which involve repeated trials, manual monitoring and follow ups, the digital therapeutic application is able to continuously monitor and provide recommendations including selections of weight loss medications, as well as optimized dosage, frequency, and timing of administration, among others. By reducing reliance on manual evaluations and sequential trials, the digital therapeutic application is able to execute data-driven outputs more accurately and quickly.

Furthermore, the digital therapeutic application can provide a number of visualizations to facilitate medication adherence. The digital therapeutic application provides visual motivators to encourage adherence to the medication prescription. The digital therapeutic application can generate a simulation that represents an experience of the user as the user takes the medication. For instance, based on the physiological measurements, the simulation can identify a cause of dropout, probability of side effects, or predicted amount of weight loss, among others, and represent this information over a period of time on a graph. These visualizations serve as persuasive tools to motivate users to take the medication prescription and also prepare for predicted side effects. The likelihood of side effects as well as the predicted amount of weight loss is continuously updated to reflect real-time data received by the digital therapeutic application to ensure that the information provided to the user remains accurate and effective.

Accordingly, the digital therapeutic application addresses the lack of accurate, personalized integration of real-world data by providing a model that dynamically predicts individual responses to weight loss medications. The application offers a precise and tailored approach to weight loss that aligns with the physiological profiles of each patient to enhance overall weight loss while minimizing side effects. Through this integration of digital and pharmacological solutions, the treatment of obesity is greatly improved, leading to superior outcomes and enhancing overall patient care.

Aspects of the present disclosure are directed towards systems and methods for selecting a weight loss medication for an obesity condition in a user. One or more processors may receive one or more physiological measurements of the user. The one or more processors can apply the one or more physiological measurements to a machine learning model. The machine learning model can be trained using a plurality of examples, each example comprising one or more sample physiological measurements of a sample user and a corresponding sample weight loss medication administered to the sample user. The one or more processors can generate, based on applying the one or more physiological measurements to the machine learning model, a metric indicating an expected outcome associated with a weight loss medication for the user. The one or more processors can provide to a user device associated with the user, a message indicating at least one of a selection of a weight loss medication based on the metric indicating the expected outcome or the expected outcome associated with the weight loss medication.

In various implementations, the one or more processors select the weight loss medication from a plurality of weight loss medications based on a plurality of expected outcomes associated with the plurality of weight loss medications. In various implementations, the one or more physiological measurements comprise at least one of: body-mass index, weight, blood pressure, heart rate, smoking status, glucose excretion, comprehensive metabolic panel, complete blood count, lipase levels, thyroid panel, magnesium level, HgAlc, fasting glucose, energy expenditure, physical activity, hormone levels, body weight, body fat percentage, a genetic marker, an evaluation of gut microbiome, or energy intake. The machine learning model comprises one or more corresponding weights to generate one or more values, the one or more corresponding weights comprising at least one of a binary weight or a continuous weight. The one or more processors can then generate the metric based on the one or more values. The weight loss medication can be selected from a GLP-1 receptor agonist or a GIP receptor agonist. The GLP-1 receptor agonist can be selected from one or more of semaglutide, liraglutide, exenatide, and dulaglutide, and wherein the GIP receptor agonist comprises tirzepatide.

In various implementations, the expected outcome further includes at least one administration parameter for the weight loss medication, the administration parameter comprising at least one of a dosage of the weight loss medication, a timing of administration of the weight loss medication, a frequency of administration, a route of administration, a dose escalation protocol, circumstances of administration, or any combination thereof. In various implementations, the expected outcome further includes identifying at least one of a therapy discontinuation, side effects, or therapeutic efficacy. In various implementations, the side effects are selected from nausea, vomiting, diarrhea, early satiety, loss of appetite, anorexia, dizziness, increased heart rate, indigestion, headache, hypoglycemia, calculus of a kidney or ureter, pancreatitis, diabetic retinopathy, depression, suicidal ideation or attempts, pain in abdomen, acute kidney injury, muscle wasting and atrophy, constipation, or any combination thereof. The one or more processors can, to generate the metric, generate a plurality of metrics for a plurality of expected outcome parameters based on applying the one or more physiological measurements to the machine learning model.

In various implementations, the expected outcome further includes at least one expected outcome parameter, the expected outcome parameter comprising at least one of a timing of discontinuation, a cause of discontinuation, a probability of discontinuation, a discontinuation mitigation, or any combination thereof. In various implementations, the expected outcome further comprises at least one expected outcome parameter, the expected outcome parameter comprising at least one of timing of onset of the side effects, duration of the side effects, probability of the side effects, side effect mitigations, or any combination thereof. In various implementations, the expected outcome further includes at least one expected outcome parameter, the expected outcome parameter comprising at least one of timing of onset of the side effects, duration of the side effects, probability of the side effects, side effect mitigations, or any combination thereof. In various implementations, the expected outcome further includes an expected outcome parameter, the expected outcome parameter comprising at least one of weight loss, fat loss, fasting blood glucose, cholesterol levels, hormone levels, duration of fat loss, risk of weight regain, a change in body mass index, or any combination thereof.

In various implementations, the one or more processors generate a simulation identifying a plurality of expected outcomes over a corresponding plurality of timepoints. The simulation may include a representation of the plurality of expected outcomes over the corresponding plurality of timepoints, wherein the representation comprises at least one of a timeline, a graph, a video, an audio, or an avatar. In various implementations, the simulation can identify the plurality of expected outcomes including at least one expected outcome parameter, the expected outcome parameter comprising at least one of a timing of discontinuation, a cause of discontinuation, a probability of discontinuation, a discontinuation mitigation (also herein referred to as reduction, alleviation, minimization, or prevention, among others), or any combination thereof. The simulation can identify the plurality of expected outcomes including at least one expected outcome parameter, the expected outcome parameter comprising at least one of timing of onset of side effects, duration of side effects, probability of side effects, side effect mitigations, or any combination thereof. The simulation can identify the plurality of expected outcomes including an expected outcome parameter, the expected outcome parameter comprising at least one of weight loss, fat loss, fasting blood glucose, cholesterol levels, hormone levels, duration of fat loss, risk of weight regain, a change in body mass index, or any combination thereof.

In various implementations, the user has a BMI greater than 25, a body fat percentage greater than 20%, Type I Diabetes, Type II Diabetes, or nonalcoholic steatohepatitis (NASH). The one or more processors can provide the message to the user device or a user's clinician. The one or more processors can obtain the one or more physiological measurements of the user by at least one of the user device or an instrumentation device on the user. The one or more processors can, to generate the metric indicating the expected outcome, determine the metric based on at least one of: (i) an average of the plurality of examples, (ii) a weighted combination of the plurality of examples, or (iii) a comparison with a dataset comprised of the plurality of examples. In various implementations, the one or more processors to receive the one or more physiological measurements can receive, over a time period, the one or more physiological measurements from at least one of the user device or an instrumentation device. To apply the machine learning model, the one or more processors can apply the one or more physiological measurements to the machine learning model, responsive to elapsing of the time period. To generate the metric, the one or more processors can generate the metric indicating the expected outcome associated with the weight loss medication for a subsequent time period.

In various implementations, the one or more processors receive one or more subsequent physiological measurements of the user. The one or more processors can apply, responsive to receipt of the one or more subsequent physiological measurements, the one or more subsequent physiological measurements to the machine learning model. The one or more processors can generate, based on applying the one or more subsequent physiological measurements to the machine learning model, a subsequent metric indicating a subsequent expected outcome associated with a subsequent weight loss medication for the user. The one or more processors can provide, to the user device within a defined time period relative to the receipt of the one or more subsequent physiological measurements, a subsequent message indicating at least one of a) a selection of the weight loss medication or a subsequent weight loss medication based on the subsequent metric indicating the subsequent expected outcome, or b) the subsequent expected outcome associated with the subsequent weight loss medication. The defined time period can range between 1 second to 1 hour.

### BRIEF DESCRIPTION OF THE DRAWINGS

The foregoing and other objects, aspects, features, and advantages of the disclosure will become more apparent and better understood by referring to the following description taken in conjunction with the accompanying drawings, in which:
FIG. 1 depicts a block diagram of a system for selecting a medication for users in accordance with an illustrative embodiment.
FIG. 2 depicts a block diagram for a process to receive measurements from a user, in accordance with an illustrative embodiment.
FIG. 3 depicts a block diagram for a process of generating a metric using a machine learning (ML) model in accordance with an illustrative embodiment.
FIG. 4 depicts a block diagram to provide simulations and a selected medication to a user, in accordance with an illustrative embodiment in accordance with an illustrative embodiment.
FIGs. 5A and 5B depict screenshots of sets of example user interfaces for selecting the medication, in accordance with an illustrative embodiment.
FIGs. 6A and 6B depict screenshots of sets of example user interfaces for selecting the medication, in accordance with an illustrative embodiment.
FIG. 7 depicts screenshots of sets of example user interfaces for selecting the medication, in accordance with an illustrative embodiment.
FIG. 8 depicts screenshots of sets of example user interfaces for notifying of side effects, in accordance with an illustrative embodiment.
FIG. 9 depicts screenshots of sets of example user interfaces for notifying of side effects, in accordance with an illustrative embodiment.
FIG. 10 depicts screenshots of sets of example user interfaces for showing an expected outcome, in accordance with an illustrative embodiment
FIG. 11 depicts screenshots of sets of example user interfaces for selecting the medication, in accordance with an illustrative embodiment.
FIG. 12 depicts a flow diagram of a method for selecting a medication for the user to address obesity in accordance with an illustrative embodiment.
FIG. 13 is a block diagram of a server system and a client computer system in accordance with an illustrative embodiment.

### DETAILED DESCRIPTION

For purposes of reading the description of the various embodiments below, the following enumeration of the sections of the specification and their respective contents may be helpful:

Section A describes systems and methods for selecting a medication to address an obesity condition in a user.

Section B describes a network and computing environment which may be useful for practicing embodiments described herein.

### A. Systems and Methods for Selecting a Medication to Address an Obesity Condition in a User

Presented herein are systems and methods for selecting weight loss medications for users with an obesity condition. The digital therapeutic application described herein can receive physiological measurement of the user. The physiological measurements may include values input by the user, testing results, or wearable technology data, among others. Once the physiological measurements are received, the application can apply the physiological measurements to a machine learning model to select a weight loss medication for the user based on expected outcomes. The expected outcomes may include result, effect, and impact, such as a timing of an onset of side effects, when the user is administered with a particular weight loss medication, among others.

The machine learning model may be trained on training data derived from real-world data including the impact of specific weight loss medications and prescriptions on individuals with a variety of physiological measurements of sample subjects. As a result of the training, the machine learning model may assign weights for each physiological measurement. The weights may be embedded with the patterns (or latent features) apparent in the training data. In addition, the application can also generate a simulation based on the physiological measurements to visualize an impact of the weight loss medication on the user, such as side effects and weight loss. As the application receives more data from, for example, the wearable technology, the predicted weight loss and other such factors can be updated to reflect the real-time data.

By using the machine learning model, the application can generate a metric indicative of an expected outcome associated with the selected weight loss medication for the user. To select the weight loss medication, the application may generate a set of expected outcomes, and select the medication with the least amount of side effects and most desirable healthy outcomes. The expected outcome can include, for example, timing of onset of side effects, fat loss, duration of fat loss, or cholesterol levels, blood glucose, insulin levels, among others. The application can take into account the physiological measurements to generate the metric based on a predicted result and impact of the selected weight loss medication on the user. The application can then provide a message to the user indicating at least one of a selection of a weight loss medication based on the metric indicating the expected outcome or the expected outcome associated with the weight loss medication. The message may also include a visualization of the expected outcome, such as in a form of a graph, avatar, timeline, video, or audio.

In this manner, the application may leverage real-world data to identify an optimal weight loss medication based on the physiological measurements of the user to promote weight loss in the user. By taking into account a wide variety of physiological measurements, the application can select a weight loss medication, and generate expected outcomes tailored to the user as a result of taking the weight loss medication. As the application receives subsequent physiological measurements, the application may update the weight loss medication and associated expected outcome. For example, as the user continues to lose weight, a dosage and frequency of the weight loss medication may be adjusted by the application as well as the expected outcome. The application can blend pharmacotherapy and digital therapy solutions to facilitate weight loss in users, while avoiding prescribing weight loss medications based on trial and error.

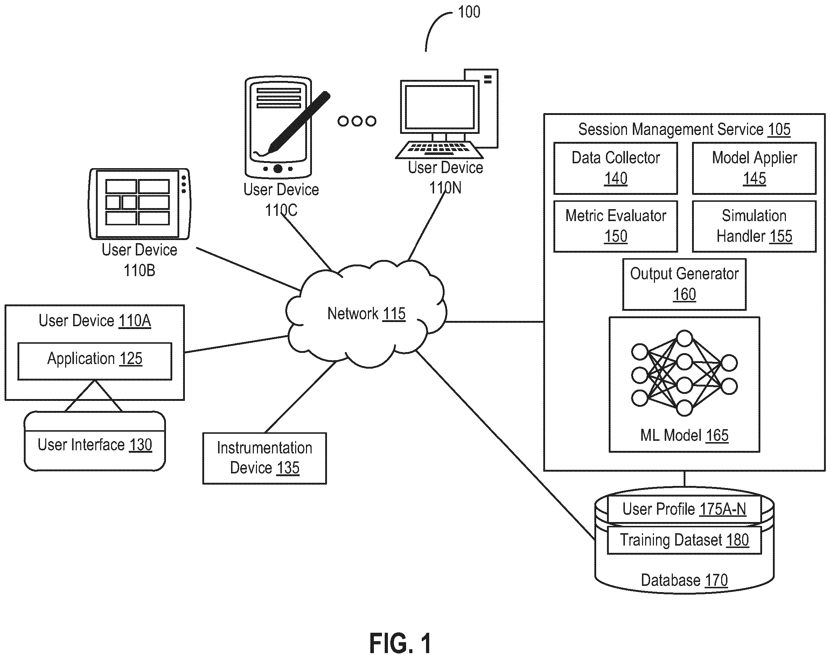

Referring now to FIG. 1, depicted is a block diagram of a system 100 for presenting interactive sessions to address obesity in users. In an overview, the system 100 may include at least one session management service 105, a set of user devices 110A-N (hereinafter generally referred to as user devices 110), and an instrumentation device 135 communicatively coupled with one another via at least one network 115. At least one user device 110 (e.g., the first user device 110A as depicted) may include at least one application 125. The application 125 may include or provide at least one user interface 130. The session management service 105 may include at least data collector 140, model applier 145, metric evaluator 150, simulation handler 155, output generator 160, and at least one machine learning (ML) model 165, among others. The session management service 105 may include or have access to at least one database 170. The database 170 may store, maintain, or otherwise include one or more user profiles 175A-N (hereinafter generally referred to as user profiles 175) and a training dataset 180. The functionality of the application 125 may be performed in part on the session management service 105. Conversely, the functionality of the application 125 may also incorporate operations performed on the session management service 105. Collectively, the user device 110 and the session management service 105 may be part of a computing system to provide the application 125.

In further detail, the session management service 105 may (sometimes herein generally referred to as a service) be any computing device comprising one or more processors coupled with memory and software and capable of performing the various processes and tasks described herein. The session management service 105 may be in communication with the one or more user devices 110 and the database 170 via the network 115. The session management service 105 may be situated, located, or otherwise associated with at least one server group. The server group may correspond to a data center, a branch office, or a site at which one or more servers corresponding to the session management service 105 is situated. The session management service 105 may be situated, located, or otherwise associated with one or more of the user devices 110. Some components of the session management service 105 may be located within the server group, and some may be located within the client device. For example, the session management service 105 may operate or be situated on the user device 110, and the ML model 165 may operate or be situated on the server group.

Within the session management service 105, the data collector 140 may present to a user by the application 125 on respective user devices 110 data to input. The data collector 140 can then collect the data provided by the user, and the model applier 145 can receive the data. The model applier 145 can apply the data from the user to a machine learning model (e.g., the ML model 165) to analyze the data to determine metrics for weight loss medications. The simulation handler 155 can simulate various outcomes of the weight loss medications for the user based on the data to provide visual outputs to the user. The output generator 160 can select a medication based on the metrics and provide a message to the user alongside the medication and the visual outputs.

The ML model 165 (sometimes referred herein as a machine learning (ML) model) can be used to generate a set of expected outcomes for a set of weight loss medications, and select a weight loss medication based on the set of expected outcomes. The architecture for the machine learning model can include, for example, a deep learning neural network (e.g., convolutional neural network architecture, a residual network, or a transformer-based architecture), a regression model (e.g., linear or logistic regression model), a random forest, a gradient boosting, a K-neighbors classifier and/or regressor, a support vector machine (SVM), a clustering algorithm (e.g., k-nearest neighbors), or a Naive Bayesian model, among others and be supervised, unsupervised, or self-supervised.

In general, the ML model 165 may have at least one input and output. The input and output may be related via a set of weights. The input may be data from the user, among others, while the output may include at least one or more medication selections, expected outcomes, and visual outputs, among others. The visual outputs can include a timeline, a graph, a video, an audio, or an avatar. The ML model 165 can be trained using the training dataset 180. The training dataset 180 may include a set of examples representing subjects that have been treated with a weight loss therapy. Each example can include an input (e.g., weight-loss medication, physiological measurements, selected medication) and outcomes (e.g., weight loss, side effects, discontinuation). In some embodiments, the session management service 105 may maintain a set of ML models 165, each for a particular user or clinician examining the user.

The user device 110 (sometimes herein referred to as an end user computing device or client device) may be any computing device comprising one or more processors coupled with memory and software and capable of performing the various processes and tasks described herein. In some embodiments, the user device 110 may be associated with a user taking weight loss medication. In some embodiments, the user device 110 may be associated with a clinician of an individual (e.g., to prescribe weight loss medication to the individual). The user device 110 may be in communication with the session management service 105, the instrumentation device 135, and the database 170 via the network 115. The user device 110 may be a smartphone, other mobile phone, tablet computer, a wearable device (e.g., smart watch, eyeglasses), or laptop computer. The user device 110 may be used to access the application 125. In some embodiments, the application 125 may be downloaded and installed on the user device 110 (e.g., via a digital distribution platform). In some embodiments, the application 125 may be a web application with resources accessible via the network 115.

The instrumentation device 135 (sometimes herein referred to as a wearable technology, wearable device, or a device) may be any computing device capable of collecting measurements from the user. The instrumentation device 135 can include fitness trackers, smart watches, heart monitors, pedometers, or glucose monitors, among others. The instrumentation device 135 may be worn by (e.g., attached to) the user to collect various data. The instrumentation data may continuously provide measurement data to the user device 110 (e.g., the application 125 on the user device 110) or the data collector 140. The instrumentation device 135 may be in communication with the session management service 105, the user device 110, and the database 170 via the network 115, among others.

The application 125 executing on the user device 110 may be a digital therapeutics application and may provide the sessions (sometimes herein referred to as a therapy session) to address obesity conditions and diseases associated with obesity conditions. The user of the application 125 may be diagnosed with, or at risk of a disease or disorder associated with an obesity condition. For example, the user can have diabetes, high blood pressure, hypercholesterolemia, fatigue, excess body fat, psychological issues, snoring, shortness of breath, or physical impairments, among others. The disease or condition may include any number of conditions that cause obesity in the user. The user can have a body weight index (BMI) greater than or equal to 25, greater than or equal to 30, greater than or equal to 32, or greater than or equal to 35, among others. The user can have a body fat percentage of greater than 20%, greater than 25%, or greater than 30%, among others. The user can have Type I diabetes, Type II diabetes, nonalcoholic steatohepatitis (NASH), or nonalcoholic fatty liver disease (NAFLD). The causes of obesity can include genetic, behavioral, environmental, physiological, and psychological factors, among others. For example, certain genetic traits can affect weight gain such as a slower metabolism and/or appetite regulation. A family history of obesity can also increase a likelihood of developing obesity based on lifestyle habits and shared genetics. In another example, metabolic conditions (e.g., diabetes), can cause weight gain such as due to increased appetite and caloric intake due to higher blood sugar levels.

Obesity can result in physical health effects, mental health effects, social and economic effects, and impact children of a patient with obesity. The physical health effects may include, for example, a cardiovascular disease (e.g., hypertension, heart disease, or stroke) or a metabolic disorder (e.g., diabetes or dyslipidemia), among others. The condition may impede or hinder social skills, such as facing discrimination or stigma in various social settings, such as the workplace or healthcare facilities.

The application 125 may be used to select a medication and provide the user with expected outcomes associated with the medication. The medication may be presented as a result of the user inputting data (e.g., physiological measurements), selecting a medication, or generating a metric (also herein referred to as measure, indicator, parameter, or criterion, among others) indicating the expected outcome, among others. The medication may be presented alongside a visual output of the expected outcome, such as a timeline showing timing onset of side effects and weight loss over time. By providing the user the digital therapeutics (e.g., the intervention) through the application 125, the adverse effects of obesity can be addressed.

The physiological measurements may include at least one or more of the user's body-mass index, weight, blood pressure, heart rate, smoking status, glucose excretion, comprehensive metabolic panel, complete blood count, lipase levels, thyroid panel, magnesium level, HgA1c, fasting glucose, energy expenditure, physical activity, hormone levels, body weight, body fat percentage, a genetic marker, an evaluation of gut microbiome, or energy intake. The expected outcome may include at least one or more of a therapy discontinuation (e.g., drop out, discontinuation of taking the medication), side effects, or therapeutic efficacy (e.g., of the medication) (also herein referred to as effectiveness, performance, or success rate, among others).

The user may be at least partially concurrently receiving a treatment to address the condition, or side effects of the condition, at least partially concurrently with the medication prescription provided by the application 125. For example, the user may be receiving treatment for diabetes. The user may be receiving a treatment at least partially concurrently with a first medication selection, a second medication selection, a third medication selection, or any combination thereof. The treatment can include a taking of a medication. The medication may be at least orally administered, intravenously administered, or topically applied. For example, for metabolic conditions (e.g., diabetes or hypothyroidism), the user may be on diabetes medications (e.g., insulin, biguanides, sulfonylureas, or meglitinides) or beta-blocker drugs (e.g., propranolol or atenolol), among others. The treatment can include blood tests, bariatric surgery, metabolic surgery, nutritional counseling, psychological counseling, or weight management programs, among others.

To address obesity, medications, such as glucagon-like peptide-1 receptor agonists (GLP-1 RA) and/or gastrointestinal inhibitory peptide (GIP), may be administered to the user to facilitate weight loss. GLP-1 RAs mimic actions of the naturally occurring hormone, GLP-1, which regulates blood sugar levels, insulin secretion, and appetite. GIP therapies are analogues of human GIP hormones that stimulate the release of insulin from the pancreas. As such, GLP-1 RAs and GIPs can increase insulin release, reduce glucagon release, and slow gastric emptying to reduce appetite and lower blood sugar levels, thereby promoting weight loss. Examples of GLP-1 RAs include semaglutide, liraglutide, exenatide, and dulaglutide. Examples of GIPs include tirzepatide. In some aspects, the medications can include any analogue of a GLP-1 RA or a GIP. The medications can include functionally equivalent peptides or therapeutic agents. Furthermore, the medications may include any medication demonstrating therapeutic efficacy for the treatment of obesity and obesity-related conditions. For example, the medication may include agents that are capable of GLP-1 or GIP agonism, or medications that are capable of inducing fat loss and/or suppressing appetite of the user.

The database 170 may store and maintain various resources and data associated with the session management service 105 and the application 125. The database 170 may include a database management system (DBMS) to arrange and organize the data maintained thereon. The database 170 may be in communication with the session management service 105 and the one or more user devices 110 via the network 115. While running various operations, the session management service 105 and the application 125 may access the database 170 to retrieve identified data therefrom. The session management service 105 and the application 125 may also write data onto the database 170 from running such operations.

Such operations may include the maintenance of the user profile 175 (sometimes herein referred to as a subject profile). The user profile 175 can include information pertaining to a condition of a user, as described herein. For example, the user profile 175 may include information related to the severity of the condition, occurrences of the condition (such as occurrences of symptoms associated with the condition affecting the cognitive functioning of the user), medications or treatments the user takes for the condition, and/or a duration of the condition, among others. The user profile 175 can be updated responsive to a schedule, periodically (e.g., daily, weekly), responsive to a change in user information (e.g., input by the user via the user interface 130 or learned from the user device 110), or responsive to a clinician (e.g., a doctor or nurse) addressing the user's condition, among others.

The user profile 175 can store and maintain information related to a user of the application 125 through user device 110. Each user profile 175 may be associated with or correspond to a respective user of the application 125. This directed approach can reduce the need for multiple communications with the user, thereby reducing bandwidth and increasing the benefit of the user-computer interaction. In some embodiments, the user profile 175 may identify or include information on a treatment regimen undertaken by the user, such as a type of treatment (e.g., therapy, pharmaceutical, or psychotherapy), duration (e.g., days, weeks, or years), and frequency (e.g., daily, weekly, quarterly, annually), among others. The user profile 175 may be stored and maintained in the database 170 using one or more files (e.g., extensible markup language (XML), comma-separated values (CSV) delimited text files, or a structured query language (SQL) file). The user profile 175 may be iteratively updated as the user provides responses, makes selections, and performs actions related to the session, the data collector 140, or the output generator 160. For example, the user profile 175 can be updated with data collected by the data collector 140.

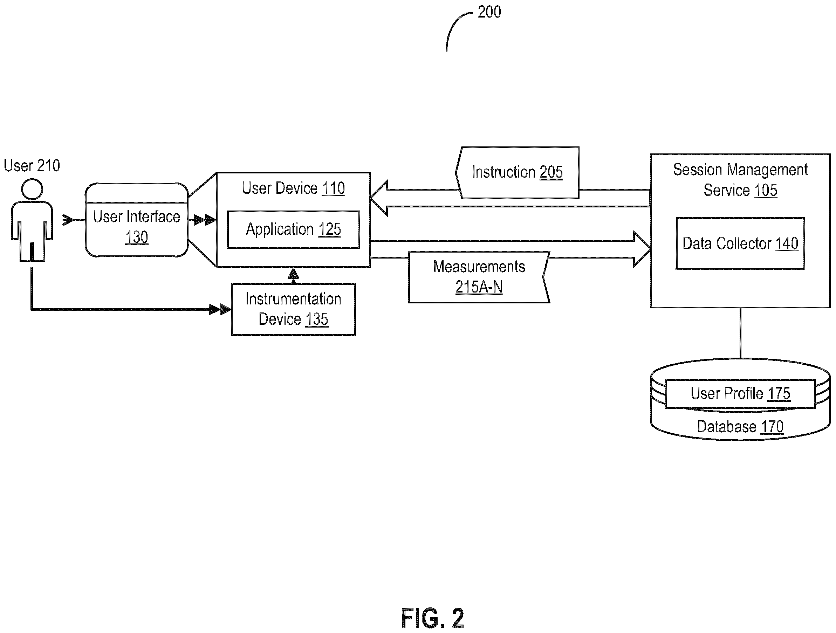

Referring now to FIG. 2, depicted are block diagrams of a process 200 to collect measurements from a user 210 to select a medication in the system 100. The process 200 may include or correspond to operations performed by the system 100 to receive and process data from users. Under the process 200, the data collector 140 executing on the session management service 105 may be in communication with the user device 110 or the instrumentation device 135 associated with at least one user 210. The user 210 may have a body-mass index (BMI) greater than 25, a body fat percentage greater than 20%, Type I diabetes, Type II diabetes, or NASH, among others. The user 210 may have an obesity condition associated with diabetes, high blood pressure, or high cholesterol, among others.

The data collector 140 may create, write, or otherwise generate one or more instructions 205 (hereinafter generally referred to as instructions 205) for at least one user 210. The instruction 205 may be to request measurements from the user 210 (or the application 125 or the instrumentation device 135). The instruction 205 may identify one or more measurement fields to be acquired via the user device 110. The one or more measurement fields may be related to physiological measurements of the user 210. The measurements may be across multiple modalities, such as numerical values, time-series data, imaging, Boolean values, or free text, among others. The one or more measurement fields may include at least one of: body mass index (BMI) (e.g., calculated by the user's height and weight measurements), weight (e.g., total mass of the user 210), blood pressure (e.g., force exerted by circulating blood upon the arterial walls), heart rate (e.g., number of times the heart beats within 60 seconds), smoking status (e.g., indication of whether the user 210 smokes), glucose excretion, comprehensive metabolic panel, complete blood count, lipase levels, thyroid panel, magnesium level, HgA1c, fasting glucose, energy expenditure, physical activity, hormone levels, body weight, body fat percentage, a genetic marker, an evaluation of gut microbiome, or energy intake, among others.

In some embodiments, the one or more measurement fields may also include at least one questionnaire. The questionnaire may be for the user 210 to indicate at least one of injuries (e.g., physical injury impacting ability of user to exercise; user recently experiencing vertigo impacting ability of user to tolerate nausea) or a side effect (e.g., negative response to a particular weight loss medication or to other non-weight loss medications) or a side effect preference (e.g., preference to limit nausea as a side effect because user is a pilot and cannot get nausea while on the job) in the user 210. For example, the questionnaire may inquire the user 210 regarding: (i) general side effects since start of the medication, (ii) an identification of symptoms (e.g., nausea, vomiting, diarrhea, early satiety, loss of appetite, anorexia, dizziness, increased heart rate, indigestion, headache, hypoglycemia, calculus of a kidney or ureter, pancreatitis, diabetic retinopathy, depression, suicidal ideation or attempts, pain in abdomen, acute kidney injury, muscle wasting, or atrophy), (iii) a start date of the side effects, (iv) a frequency of occurrence of the side effects, or (v) impact on daily life, among others. As another example, the measurement field may include questions for the user 210 to answer regarding side effects the user 210 can tolerate. The measurement field may also request the user 210 to input information on recent injuries, such as a knee or ankle injury impacting the ability of the user 210 to physically move. The questionnaires may be generated based on questions identified in the user profile 175.

In generating the instructions 205, the data collector 140 may select or identify the one or more physiological measurement fields based on at least the user profile 175. For example, the physiological measurements identified in the instructions 205 may depend on the condition of the user 210. The user profile 175 can contain conditions (e.g., Type 1 diabetes) the user has had in the past and present which can be used to select the medication, and the data collector 140 may identify blood sugar, among other measurements, in the instruction 205. In some embodiments, the instruction 205 may identify the one or more measurement fields associated with a time period. For example, the instruction 205 may define a time period for which the user 210 to input the one or more measurements fields. The time period may range between 1 day to 6 months.

With the generation, the data collector 140 may send, provide, or otherwise transmit the instruction 205 to the user device 110 or the instrumentation device 135. The transmission of the instruction 205 may be in accordance with a schedule (e.g., at an interval between 5 minutes to 2 weeks). In some embodiments, the data collector 140 may transmit the instruction 205 to the user device 110 for at least a portion of the measurements. The data collector 140 may transmit the instruction 205 to the instrumentation device 135 of another portion of the measurements. The instructions 205 may take various formats associated with the application 125. In some embodiments, the instructions 205 may be displayed, rendered, or otherwise presented via the user interface 130 of the application 125 to the user 210. The instructions 205 may take a form of a list, table, database, graph, or chart, among others displaying the one or more measurement fields for the user 210 to input. In some embodiments, the instructions 205 may include a short message service (SMS) (e.g., text message) or multimedia message service (MMS) (e.g., audio message, video message). For example, the instructions 205 may include a link to open the application 125 directing the user to input the one or more measurement fields via the user interface 130 of the application 125.

The application 125 on the user device 110 may retrieve, identify, or otherwise receive the instruction 205 from the session management service 105. Upon receipt of the instruction 205, the application 125 may parse the instruction 205 to identify the measurement fields to be provided to the session management service 105. In some embodiments, the application 125 can display, render, or otherwise present the user interface 130 using the instruction 205. The application 125 may then prompt or direct the user 210 to provide measurements 215A-N (e.g., one or more measurements herein referred to as measurements 215) for each of the one or more measurement fields. The measurements 215 can be a text string, character string, or a numerical value, among others, entered by the user 210 on the user interface 130. In some embodiments, upon receipt, the application 125 may retrieve, fetch, or otherwise identify the measurements 215 as specified by the instruction 205. The application 125 on the user device 110 may have been generating and storing the data associated with the measurements 215 from the user 210 (or the instrumentation device 135 in communication with the user device 110). In response to the instruction 205, the application 125 may fetch the measurements 215 stored on the data. With the identification, the application 125 may return, provide, or otherwise send the measurements 215 to the session management service 105.

In some embodiments, the instrumentation device 135 may retrieve, identify, or otherwise receive the instruction 205 from the session management service 105. Upon receipt, the instrumentation device 135 may parse the instruction 205 to identify the measurement fields to be provided to the session management service 105. The instrumentation device 135 may retrieve, fetch, or otherwise identify the measurements 215 as specified by the instruction 205. The instrumentation device 135 on the user device 110 may have been generating and storing the data associated with the measurements 215 from the user 210. With the identification, the instrumentation device 135 may return, provide, or otherwise send the measurements 215 to the session management service 105. In some embodiments, the instrumentation device 135 can obtain measurements 215 for the one or more measurement fields (e.g., heart rate). The instrumentation device 135 can include a wearable continuous glucose monitor, a smart watch capable of monitoring the user's heart rate and biometrics, a scale, or other technologies capable of obtaining data associated with the user's physiological health. The instrumentation device 135 can provide the measurements 215 to the data collector 140 via the application 125 in real-time (e.g., continuously).

In turn, the data collector 140 can retrieve, identify, or otherwise receive the measurements 215 from the user 210 (or the application 125 or the instrumentation device 135). With receipt, the data collector 140 can correlate or correspond each of the measurements 215 to respective measurement fields. For example, the instruction 205 may include a weight measurement field, and the data collector 140 receives a numerical value of the weight of the user 210. The data collector 140 can store the measurements 215 in the database 170 and associate the measurements 215 with the user profile 175. By storing, the data collector 140 may update the user profile 175 to add or include the measurements 215. The measurements 215 of the user 210 may be obtained by at least one of the user device 110 or the instrumentation device 135 on the user 210. The data collector 140 can receive the measurements 215 from at least one of the user device 110 or the instrumentation device 135 over a time period. In various embodiments, the data collector 140 receives one or more subsequent physiological measurements of the user 210. For example, the data collector 140 may receive measurements after providing the measurements 215 to the model applier 145. In various embodiments, the measurements 215 are continuously updated based on the instrumentation device 135 provided to the user 210.

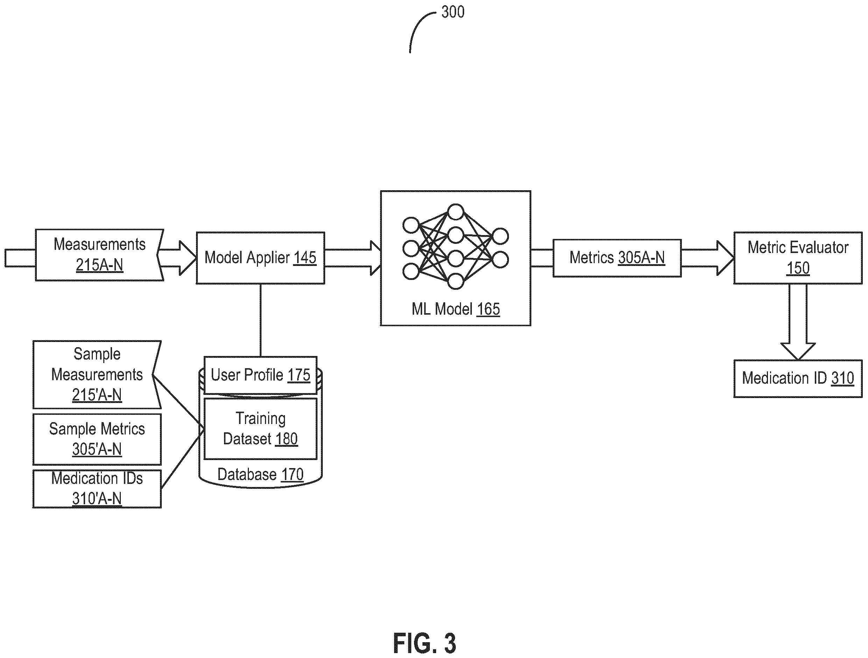

Referring now to FIG. 3, depicted are block diagrams of a process 300 to apply measurements from a user 210 to the ML model 165 to select a medication in the system 100. The process 300 may include or correspond to operations performed by the system 100 to receive and process data provided by the user 210. For the process 300, ML model 165 may be initialized, trained, and established (e.g., by the model applier 145) using the training dataset 180 on the database 170. The training dataset 180 may identify or include a set of examples. In the training dataset 170, each of the examples may include one or more sample physiological measurements of a sample user and a corresponding sample weight loss medication administered to the sample user. Each example may include sample measurements 215'A-N (herein referred to as sample measurement 215'), and sample metrics 305'A-N (herein referred to as sample metric 305'), among others.

In some embodiment, at least one example of the training dataset 180 may include medication identifiers (IDs) 310'A-N (herein referred to as medication identifiers 310') for each of the sample users. The sample users can be real-world patients. The sample users may be ages 18 and older at a time when they first begin taking a weight loss medication. The sample users may have a BMI of greater than 25 within 60 days of when they first begin taking the weight loss medication. The sample measurements 215' may also include information on each of the sample users, such as patient identification, birth year, or diagnosis (e.g., obesity, Type 1 diabetes, Type II diabetes, or NASH), among others. The sample users included in the training dataset 180 may have at least one BMI or height and weight measurement prior to starting the weight loss medication, and at least one BMI or height and weight measurement following starting the weight loss medication.

The sample measurements 215' may include corresponding physiological measurements 215 including at least one of body-mass index, weight, blood pressure, heart rate, smoking status, glucose excretion (as measured by blood or urine glucose levels), comprehensive metabolic panel (e.g.,: kidney and liver function, blood protein levels, blood electrolyte concentrations, etc.), complete blood count (e.g.,: red and white blood cell counts), lipase levels, thyroid panel (measurements for thyroid stimulating hormone, thyroxine, triiodothyronine, thyroid antibodies), magnesium level, HgAlc (average amount of sugar in blood over a specified timer period), fasting glucose, energy expenditure (as measured by caloric expenditure), physical activity, hormone levels (e.g.,: testosterone, estrogen, progesterone, etc.), body weight, body fat percentage, a genetic marker (e.g.,: genetic markers for obesity, diabetes, genetic markers related to metabolic conditions etc.), an evaluation of gut microbiome (as evaluated through a fecal panel), or energy intake (calorie, fat, protein, and carbohydrate intake). Each of the sample measurements 215' can be associated with a sample user over a given time period.

The sample metrics 305' may include at least one of therapy discontinuation, side effects, or therapeutic efficacy associated with the sample user. For example, the sample metrics 305' include International Classification of Diseases, 10^{th} revision (ICD-10) diagnosis codes for at least 20 side effects (e.g., vomiting, diarrhea, headache), lab results (e.g., complete blood count, comprehensive metabolic panel), total cholesterol, lipase, thyroid panel, magnesium, HgA1c, drop out time (e.g., stopping the weight loss medication), and available vital measurements (e.g., height, weight, smoking status). The sample metrics 305' may be associated with the sample user. The sample metrics 305' may include a set of administration parameters for the weight loss medication such as a dosage of the weight loss medication, a timing of administration of the weight loss medication (e.g.,: morning or night-time administration), a frequency of administration (e.g.,: daily, weekly, monthly administration), a route of administration (intravenously, subcutaneously, intramuscularly), a dose escalation protocol (increased or decreased dosages over time), or circumstances of administration (e.g.,: combination with other drugs or therapies), among others.

The sample metrics 305' may include information on the side effects of the medication such as timing of onset of the side effects, duration of the side effects, probability of the side effects, or side effect mitigations, among others. The sample metrics 305' may include information related to the potential discontinuation of the therapy such as timing of discontinuation (length of time on the medication until therapy discontinuation), a cause of discontinuation, a probability of discontinuation (likelihood that the user will discontinue therapy for a given time period), or a discontinuation mitigation (e.g., psychotherapy, drug side-effect mitigation measures), among others. The sample metrics 305' may include information on the efficacy of the therapy, such as weight loss, fat loss, fasting blood glucose, cholesterol levels, hormone levels, duration of fat loss, a change in body mass index, or risk of weight regain, among others.

Each of the sample measurements 215' and the sample metrics 305' for the sample users may be associated with the medication identifiers 310'. The medication identifiers 310' can identify which weight loss medication the sample user is and/or was taking. The medication identifiers 310' can include identifiers for at least one of dulaglutide, exenatide, semaglutide, liraglutide, lixisenatide, or tirzepatide. In some embodiments, the medication identifiers 310' can further indicate at least one of a dosage of the weight loss medication, a timing of administration of the weight loss medication, a frequency of administration, a route of administration, a dose escalation protocol, circumstances of administration, or any combination thereof.

To initialize, the model applier 145 may set values of the set of weights in the ML model 165 to starting values (e.g., random or defined values). To train, the model applier 145 may input, feed, or otherwise apply the sample measurements 215' and the medication identifiers 310' and compare an output metric of the ML model 165 with the sample metrics 305' for each of the sample users. Based on the comparison, the model applier 145 may determine a loss metric in accordance with a loss function (e.g., a mean squared error, cross-entropy loss, hinge loss, or Huber loss). Using the loss metric, the model applier 145 can update the one or more weights of the ML model 165. The updating of the weights may be in accordance with a back propagation and optimization function (sometimes referred to herein as an objective function) with one or more parameters (e.g., learning rate, momentum, weight decay, and number of iterations). The optimization function may define one or more parameters at which the weights of the ML model 165 are to be updated. The optimization function may be in accordance with stochastic gradient descent, and may include, for example, an adaptive moment estimation (Adam), implicit update (ISGD), and adaptive gradient algorithm (AdaGrad), among others. The ML model 165 may be iteratively updated until convergence.

With the establishment of the ML model 165, the model applier 145 can feed, provide, or otherwise apply the measurements 215 to the ML model 165. In applying, the model applier 145 may process the measurements 215 in accordance with the set of weights of the ML model 165. In some embodiments, the set of weights of the ML model 165 correspond to the measurements 215. For example, for each of the measurements 215, the ML model 165 includes a set of corresponding weights. In some embodiments, the set of corresponding weights can include at least one of a binary weight (e.g., 0 or 1) or a continuous weight (e.g. numerical values ranging between -100 and 100). For example, a fat percentage of the user 210 may be weighted higher than a weight of the user 210. Based on the set of corresponding weights, the ML model 165 generates one or more values. The one or more values may be a function of the measurements 215 with their corresponding weights.

In some embodiments, the ML model 165 may be particular to a specific user (e.g., the user 210) of the application 125. The ML model 165 may have a bias to the set of weights in accordance with the machine learning architecture to provide higher or lower values, depending on measurements for the user 210. The biasing may be performed using re-weighting (e.g., manual setting of values for weights), data augmentation, regularization, or sampling, among other techniques. The weights of the ML model 165 may be assigned or set in accordance with the user profile 175 for the specific user 210. For example, the user profile 175 may indicate that the user 210 has experienced particular types of side effects (e.g., dizziness or nausea) to a particular drug, whereas the user 210 has had no issues with another medication. The weights of the ML model 165 may be updated or assigned to have a bias to generate outputs indicating higher values for expected outcomes for the user 175 to one drug relative to the other medication. The weights of the ML model 165 may be assigned or set by a clinician (e.g., physician examining the user 210) using a user interface provided by the session management service 105. For instance, a physician may have two individuals. One individual may relay previous experience with side effects to a pre-disposition to a particular medication. Another individual may indicate no concerns with the same medication but has had issues with adherence to other medications with a higher frequency administration due to a busy work schedule. One instance of the ML model 165 for the first individual may be set with a bias to output higher probability of factors for the medication identified by the individual. Another instance of the ML model 165 for the second individual may be set with higher values for weights related to side effects known to lead to low adherence (e.g., higher frequency of administration). As a result, the ML models 165 for these two individuals may yield different expected outcomes for these two individuals even with same or similar measurement data.

In some embodiments, the model applier 145 may apply the measurements 215 responsive to elapsing of the time period as defined for the instruction 205. The model applier 145 may wait to apply the measurements 215 received over the time period by the user device 110 or the instrumentation device 135 as defined by the instructions 205. Upon elapsing of the time period, the model applier 145 may apply the measurements 215 to the ML model 165. In some embodiments, the model applier 145 may apply the one or more subsequent physiological measurements to the ML model 165 upon receipt of the one or more subsequent physiological measurements by the data collector 140.

Based on the application of the measurements 215 to the ML model 165, the ML model 165 may calculate, determine, or otherwise generate at least one metric 305A-N (hereinafter generally referred to as metrics 305). The metric 305 may identify or indicate an expected outcome associated with a weight loss medication of the user 210. The metric 305 may be for a subsequent time period relative to the acquisition of the measurements 215. The time period for the metrics 305 may be in the future relative to the time period for the measurements 215 and may have any range between 1 day to 6 months. The expected outcome for the metrics 305 may identify or include at least one administration parameter for the weight loss medication. The administration parameter can include at least one of a dosage of the weight loss medication, a timing of administration of the weight loss medication, a frequency of administration, a route of administration, a dose escalation protocol, circumstances of administration, or any combination thereof.

In some embodiments, the ML model 165 may determine the metric 305 based on at least one of an average of the set of examples, a weighted combination of the set of examples, or a comparison with the training dataset 180 including the set of examples. For example, the ML model 165 can determine the metric 305 based on an average of the sample measurements 215' for each of the medication identifiers 310'. As another example, the ML model 165 can determine the metric 305 based on comparing information of the user 210 alongside the measurements 215 with the sample measurements 215' for each of the sample users. For example, the ML model 165 may determine the sample user with the highest similarity to the user 210 (e.g., weight, height, side effect preferences, weight loss goals, medication dosage), and generate the metric 305 based on the sample metric 305' of the sample user with the highest similarity. The ML model 165 may also determine the metric 305 based on a response of the user 210 to the side effect questionnaire. Based on the questionnaire, the ML model 165 may adjust the metric 305 responsive to side effects the user 210 desires to avoid, such as nausea.

In some embodiments, the ML model 165 may calculate, determine, or otherwise generate the set of metrics 305 for the corresponding set of weight loss medications. The set of weight loss medications can be selected from a GLP-1 receptor agonist or a GIP receptor agonist. The GLP-1 receptor agonist can be selected from one or more of semaglutide, liraglutide, exenatide, and dulaglutide while the GIP receptor agonist includes tirzepatide. In some embodiments, the medications can include any analogue of a GLP-1 RA or a GIP. The medications can include functionally equivalent peptides or therapeutic agents. Furthermore, the medications may include any medication demonstrating therapeutic efficacy for the treatment of obesity and obesity-related conditions. For example, the medication may include agents that are capable of GLP-1 or GIP agonism, or medications that are capable of inducing fat loss and/or suppressing appetite of the user. Each metric 305 may identify or indicate an expected outcome associated with a weight loss medication of the user 210. The expected outcome can identify or include at least one administration parameter for the weight loss medication.

In some embodiments, the expected outcome for the metric 305 may include identifying at least one of a therapy discontinuation, side effects, or therapeutic efficacy. The side effects can be selected from nausea, vomiting, diarrhea, early satiety, loss of appetite, anorexia, dizziness, increased heart rate, indigestion, headache, hypoglycemia, calculus of a kidney or ureter, pancreatitis, diabetic retinopathy, depression, suicidal ideation or attempts, pain in abdomen, acute kidney injury, muscle wasting and atrophy, constipation, or any combination thereof. The expected outcome may identify side effects associated with at least one of the medication and the administration parameter for the user 210.

In some embodiments, the model applier 145 may generate a set of the metric 305 for a set of expected outcome parameters based on applying the measurements 215 to the ML model 165. The expected outcome may include at least one expected outcome parameter. The expected outcome parameter can include at least one of a timing of discontinuation, a cause of discontinuation, a probability of discontinuation, a discontinuation mitigation, or any combination thereof. For example, the expected outcome can include expected outcome parameters associated with therapy discontinuation. The expected outcome parameter can also include at least one of a timing of onset of the side effects, duration of the side effects, probability of the side effects, side effect mitigations, or any combination thereof.

In some embodiments, the expected outcome for the metrics 305 can include parameters associated with the side effects. The expected outcome parameter can also include at least one of weight loss, fat loss, fasting blood glucose, cholesterol levels, hormone levels, duration of fat loss, risk of weight regain, a change in body mass index, or any combination thereof. The expected outcome can include parameters associated with therapeutic efficacy of the weight loss medication. parameters. For example, the model applier 145 can generate the metric 305 for timing of onset of the side effects and duration of the side effects for each of the weight loss medications. In another example, the model applier 145 can generate the metric 305 identifying a predicted change (e.g., increase or decrease) in body-mass index below a certain threshold (e.g., 25-30).

Using the metric 305, the metric evaluator 150 can identify or select at least one medication identifier 310 from a set of medication identifiers 310. The set of medication identifiers 310 can include the weight loss medications. In some embodiments, the metric evaluator 150 may select the weight loss medication for the user 210 from the set of weight loss medications. The medication identifier 310 may indicate a selected medication for the user 210 based on the metric 305. In some embodiments, the medication identifier 310 may include administration parameters such as administration route and frequency. In some embodiments, the metric evaluator 150 may select at least one of the weight loss medications based on a comparison between the metric 305 for each weight loss medication and at least one threshold. The threshold may delineate, define, or otherwise identify a value for the metric 305 at which to select the corresponding weight loss medication. If the metric 305 satisfies (e.g., greater than or equal to) the threshold, the metric evaluator 150 may select the corresponding weight loss medication for the user 210. Otherwise, if the metric 305 does not satisfy (e.g., less than) the threshold, the metric evaluator 150 may exclude the corresponding weight loss medication for the user 210.

In some embodiments, the metric evaluator 150 may select the weight loss medication for the user 210 from the set of weight loss medications as a function of set of metrics 305. For example, the metric evaluator 150 may select and generate the medication identifier 310 based on the weight loss medication with the lowest probability of discontinuation, the lowest probability of side effects, and the highest fat loss, among others. In some embodiments, the set of metrics 305 includes metrics 305 for a set of administration parameters for the weight loss medication. In some embodiments, the metric evaluator 150 may select the weight loss medication based on the side effects predicted to occur with the user 210. Responsive to a predicted side effect corresponding to selections on the side effect questionnaire of side effects the user 210 desires to avoid, the metric evaluator 150 selects a different weight loss medication. In some embodiments, the metric evaluator 150 selects one or more weight loss medications for the user 210 and/or the clinician of the user 210 to select from. The metric evaluator 150 may thus generate a plurality of medication identifiers 310 along with a plurality of expected outcomes associated with each of the medication identifiers 310.

In some embodiments, the metric evaluator 150 can also generate interventions to mitigate and/or address the side effects indicated by the expected outcome using the metrics 305 and/or the medication identifier 310. For example, responsive to the metric 305 indicating that nausea will occur during administration of the selected medication, the metric evaluator 150 can provide interventions, such as drinking more water and avoiding greasy foods. The metric evaluator 150 can also map the interventions based on the timing onset, duration, and probability of the side effects associated with the medication identifier 310.

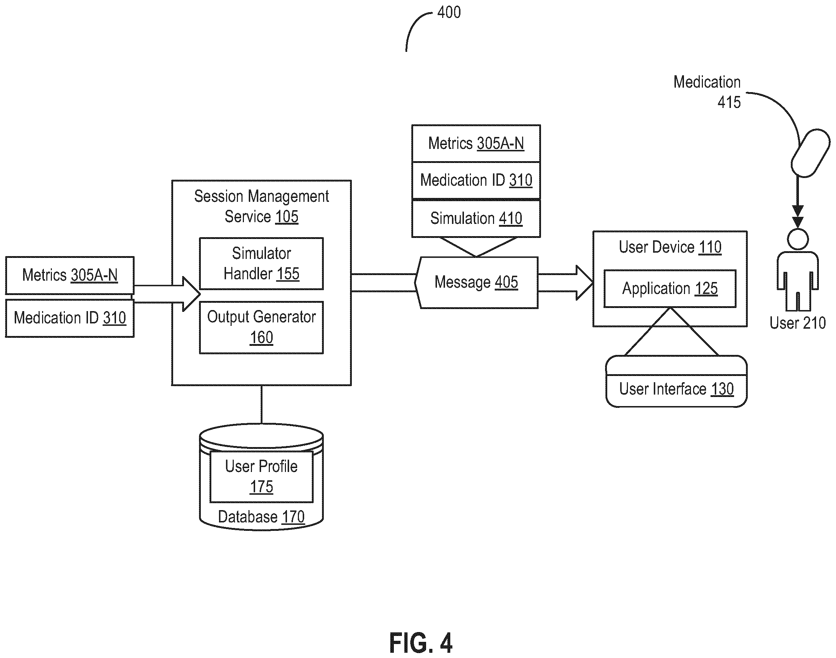

Referring now to FIG. 4, depicted are block diagrams of a process 400 to use the metric 305 and the medication identifier 310 to generate a message 405 in the system 100. The process 400 may include or correspond to operations performed by the system 100 to receive and process data provided by the user 210. Under the process 400, the simulation handler 155 executing on the session management service 105 can generate at least one simulation 410. The simulation 410 can identify a set of expected outcomes over a corresponding set of timepoints. The simulation 410 can identify expected outcome parameters over a period of time including the set of timepoints. For example, the simulation 410 can illustrate the probability of the side effects occurring over a duration of the therapy regimen (e.g., ranging between 1 week to 6 months). To generate the simulation 410, the simulation handler 155 can identify the set of expected outcomes and expected outcome parameters over a period of time by applying the measurements 215 to the ML model 165 over multiple time periods. The time periods may be increasingly further out from the present, such as 1 day to 6 months from the present. The simulation handler 155 may generate the simulation 410 for each of the set of expected outcomes.

Using the expected outcomes, the simulation handler 155 may generate the representation of the set of expected outcomes. The simulation 410 can include a representation of the set of expected outcomes. The representation can include at least one of a timeline (e.g., showing the metrics of the expected outcomes over time), a graph (e.g., expected outcomes by type), a video (e.g., showing the expected outcomes changing in value over time), an audio (e.g., a narration of the expected outcome), or an avatar (e.g. animated), among others. For example, the simulation 410 can include an avatar illustrating weight loss of the user 210 over the set of timepoints. The size of the avatar may vary based on the expected outcomes identified in the simulation 410.

In conjunction, the output generator 160 can produce, create, or otherwise generate at least one message 405. The generation of the message 405 based on the metric 305 and the medication identifier 310. The generation and provision of the message 405 may be in accordance with a schedule (e.g., at an interval between 5 minutes to 2 weeks). The message 405 may identify or indicate the expected outcome associated with the weight loss metric. In some embodiments, the output generator 160 may generate the message 405 to include the metrics 305 and the medication identifier 310, among others. The medication identifier 310 may have been selected from the set of medication identifiers 310 (corresponding to the weight loss medications) using the metrics 305.

In some embodiments, the output generator 160 can generate the message 405 to include information derived or generated from the metrics 305, the medication identifier 310, or the simulation 410, among others. The output generator 160 may generate the message 405 to include the information in accordance with a template. The template may include a set of predefined content (e.g., text, images, videos, or audio) with one or more placeholders to include the metrics 305, the medication identifier 310, or the simulation 410, among others. The message 405 can indicate the expected outcome associated with the weight loss medication. For example, the output generator 160 can extract information regarding the expected outcome to include in the message 405. In this case, the output generator 160 can include expected outcome parameters in the message 405, such as duration of fat loss associated with the medication identifier 310. The output generator 160 may insert the expected outcome parameters (e.g., duration of fat loss and medication) into the template to generate the message 405. The message 405 can include the expected outcome associated with the medication identifier 310. In some embodiments, the message 405 may include the interventions to mitigate and/or address the side effects.

In some embodiments, the output generator 160 may generate the message 405 to include the simulation 410 (e.g., the representation of the set of expected outcomes). In some embodiments, the message 405 may include the simulation 410. Following generation of the simulation 410, the simulation handler 155 can provide the simulation 410 to the output generator 160 to include in the message 405. In this case, the message 405 includes both the medication identifier 310 (e.g., the selected weight loss medication) and the simulation 410. Both the medication identifier 310 and the simulation 410 can be provided to the user device 110 and/or the clinician of the user 210. The medication identifier 310 and the simulation 410 may be displayed together or sequentially on the user device 110. With the generation of the message 405, the output generator 160 may transmit, send, or otherwise provide the user device 110 with the message 405. In some embodiments, the output generator 160 can also provide the message 405 to a computing device associated with a clinician of the user 210.

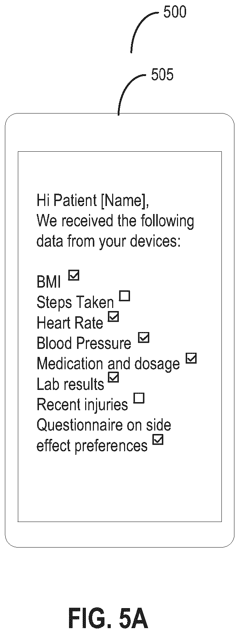

Upon receipt, the application 125 on the user device 110 can render, display, or otherwise present information based on the message 405 via the user interface 130. In some embodiments, the computing device associated with the clinician can receive the message 405 and present the information based on the message 405. The information may include or identify the metrics 305 and the medication identifier 310. For example, the application 125 can display the set of metrics 305 relating to the type of weight loss medication, likelihood of side effects, and likelihood of adherence. In some embodiments, the application 125 can display the information identifying the intervention for the user 210. In some embodiments, the application 125 may render, display, or otherwise present the simulation 410 via the user interface 130. Examples of the messages 405 presented on the user device 110 are depicted on FIGs. 5A-11.

Using the information presented via the user interface 130, the user 210 (or the clinician examining the user 210) can determine whether to take or be administered with at least one medication 415 corresponding to the medication identifier 310. The medication 415 can be provided by, for example, the clinician of the user 210. The medication 415 can include, for example, GLP-1 receptor agonist or a GIP receptor agonist (e.g., as detailed herein). The user 210 and/or the clinician of the user 210 can administer the medication 415 according to administration parameters indicated in the message 405. The user 210 may consume or take the medication 415 corresponding to the medication identifier 310.

The above-described processes can be repeated any number of times. In some embodiments, the data collector 140 may retrieve or receive subsequent measurements over a subsequent time period. The model applier 145 can generate another metric 305 for a subsequent time period by applying the subsequent measurements to the ML model 165. The metric generated following the time period may be different from a metric 305 generated prior to the time period. In some embodiments, the model applier 145 generates a subsequent metric (e.g., after generation of the metric 305) indicating a subsequent expected outcome associated with a subsequent weight loss medication for the user 210. The model applier 145 may generate the subsequent metric following a time period in which the user 210 is taking the weight loss medication. In this case, using the ML model 165, the model applier 145 may update the metric 305 based on changes represented by the subsequent physiological measurements of the user 210. Based on the subsequent physiological measurements, the subsequent expected outcome may be different from the expected outcome, such as a reduced frequency of administration of the subsequent weight loss medication. The subsequent weight loss medication may be different than the weight loss medication.
In some embodiments, the output generator 160 provides a subsequent message within a defined time period relative to receipt of the one or more subsequent physiological measurements. The output generator 160 can provide the subsequent message in responsive to receiving the subsequent metric. The subsequent message can thus indicate at least one of a) a selection of the weight loss medication or a subsequent weight loss medication based on the subsequent metric indicating the subsequent expected outcome, or b) the subsequent expected outcome associated with the subsequent weight loss medication. The defined time period can range between 1 second to 1 hour. In this case, the metric evaluator 150 may also generate a subsequent medication identifier. Thus, given subsequent physiological measurements, the ML model 165 can generate at least one subsequent metric, the metric evaluator 150 can generate a subsequent medication v, and the output generator 160 can generate a subsequent message based on the subsequent mediation ID and the subsequent metric within the defined time period. In some embodiments, the simulation handler 155 may generate a subsequent simulation to be included in the subsequent message.

In this manner, the session management service 105 may aggregate physiological measurements from the user 210 and may use the ML model 165 to determine an optimal weight loss medication that aligns with the user's unique physiological profile to enhance weight loss outcomes. By leveraging a diverse range of multi-modal data in the form of physiological parameters, the session management service 105 may algorithmically select a targeted weight loss medication and dynamically generate predictive outcomes tailored to the user. As more and more physiological measurements are received over time, the expected outcomes and by extension the recommended weight loss medication can be dynamically adjusted by the session management service 105. For instance, as the user progresses in their weight loss, the session management service 105 may automatically adjust the dosage and frequency of the medication based on updated physiological data, refining the expected outcomes accordingly. This session management service 105 may integrate pharmacotherapy and digital therapeutics to create a comprehensive, data-driven weight loss solution, thereby reducing reliance on trial-and-error medication prescribing and offering a personalized, adaptive approach to addressing obesity condition in the user 210.

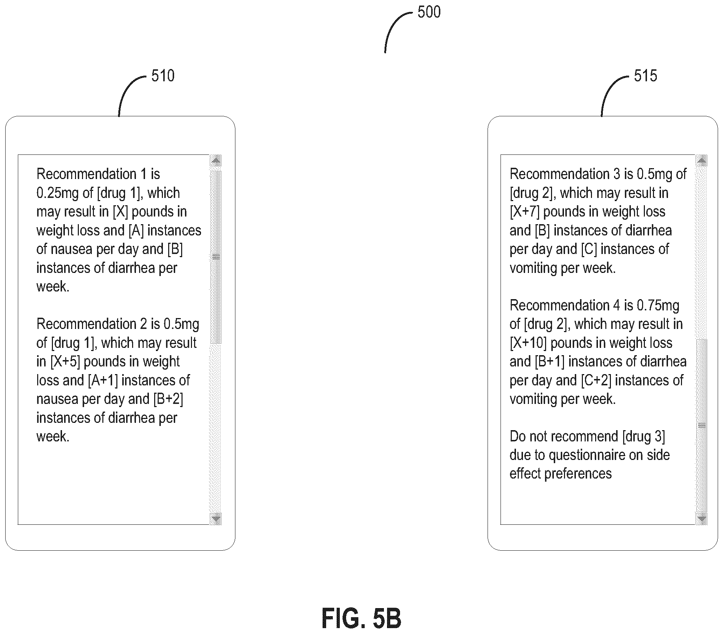

FIGs. 5A and 5B depict screenshots of a set 500 of example user interfaces for selecting the medication, in accordance with an illustrative embodiment. The user interfaces in the set 500 may be part of the application 125, and presented through the user interface 130 to the user 210 to be administered with the weight loss medication. The user interface 505 may be a prompt to the user to input values (e.g., one or more measurements) corresponding to the measurement fields displayed. The user can then input a numerical value, text string, or character string, among others, into the measurement fields. The user interface 510 and the user interface 515 may provide recommendations regarding the selected weight loss medication for the user. The recommendations may include the expected outcome, such as an amount of weight loss or the side effects associated with the weight loss medication.

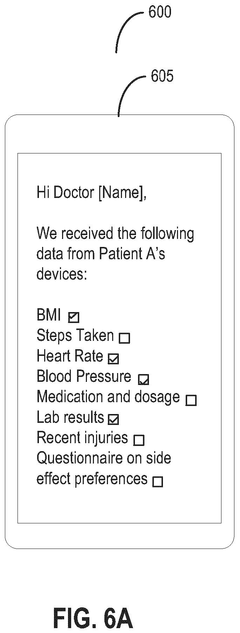

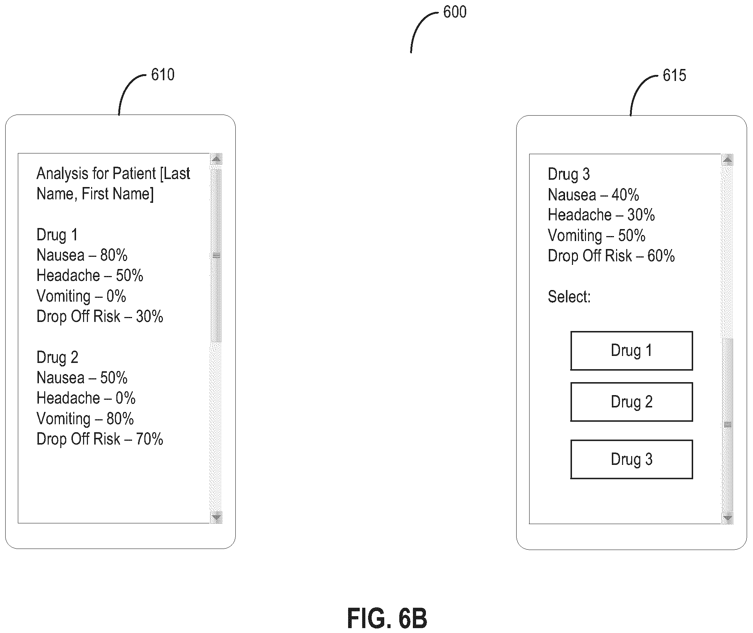

FIGs. 6A and 6B depict screenshots of a set 600 of example user interfaces for selecting the medication, in accordance with an illustrative embodiment. The user interfaces in the set 600 may be part of the application, and presented through the user interface of a computing device associated with a clinician examining a user. The user interface 605 may be a prompt to the user to input values (e.g., one or more measurements) corresponding to the measurement fields displayed. The user can then input a numerical value, text string, or character string, among others, into the measurement fields. The user interfaces 610 and 615 may display recommended weight loss medications to a user or the user's physician, and prompt the user or the user's physician to select a weight loss medication. The user interfaces 610 and 615 also display a likelihood of side effects as well as a likelihood of discontinuation (e.g., drop off risk).

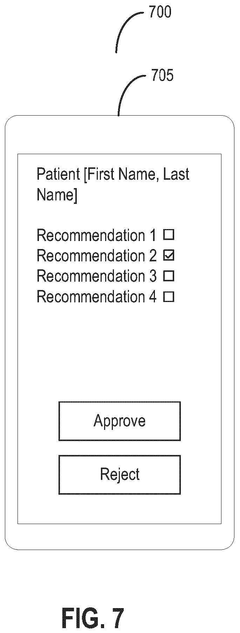

FIG. 7 depicts screenshots of a set 700 of example user interfaces for selecting the medication, in accordance with an illustrative embodiment. The user interfaces in the set 700 may be part of the application 125, and presented through the user interface 130. The user interface 705 may be shown to a clinician (e.g., doctor) of the user. The user interface 705 allows the clinician to select a recommendation (e.g., a particular drug and administration parameters) to provide to the user. For instance, the selected recommendation may be for the individual to be administered with semaglutide twice per day over a week. In some aspects, the user interface 705 may be shown to the user and allow the user to select a recommendation.

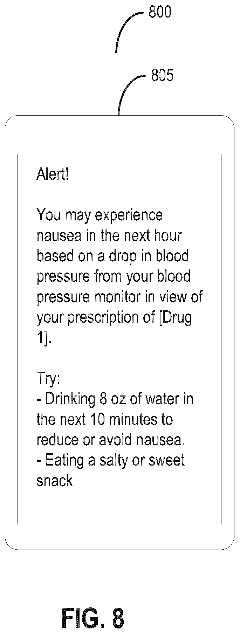

FIG. 8 depicts screenshots of a set 800 of example user interfaces for selecting the medication, in accordance with an illustrative embodiment. The user interfaces in the set 800 may be part of the application 125, and presented through the user interface 130. The user interface 805 displays a notification to the user indicating an onset of side effects associated with the weight loss medication. The notification may be generated based on wearable technology of the user. The notification may identify an action to be taken to prevent or lessen the side effects.

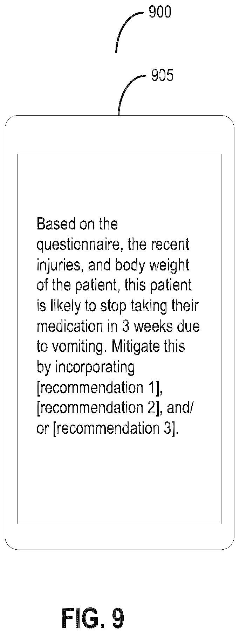

FIG. 9 depicts screenshots of a set 900 of example user interfaces for selecting the medication, in accordance with an illustrative embodiment. The user interfaces in the set 900 may be part of the application 125, and presented through the user interface 130. The user interface 905 may be presented to the clinician of the user, and provide a recommendation to mitigate drop off as well as a predicted time of discontinuation of the user to stop taking the weight loss medication.

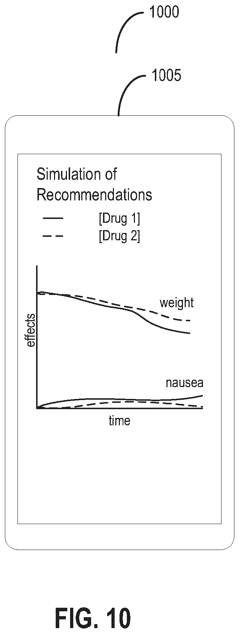

FIG. 10 depicts screenshots of a set 1000 of example user interfaces for selecting the medication, in accordance with an illustrative embodiment. The user interfaces in the set 1000 may be part of the application 125, and presented through the user interface 130. The user interface 1005 displays a simulation of the weight loss medication recommendations. For example, the simulation includes a graph displaying both weight loss as well as side effects over time. The graph can compare multiple weight loss medication recommendations.

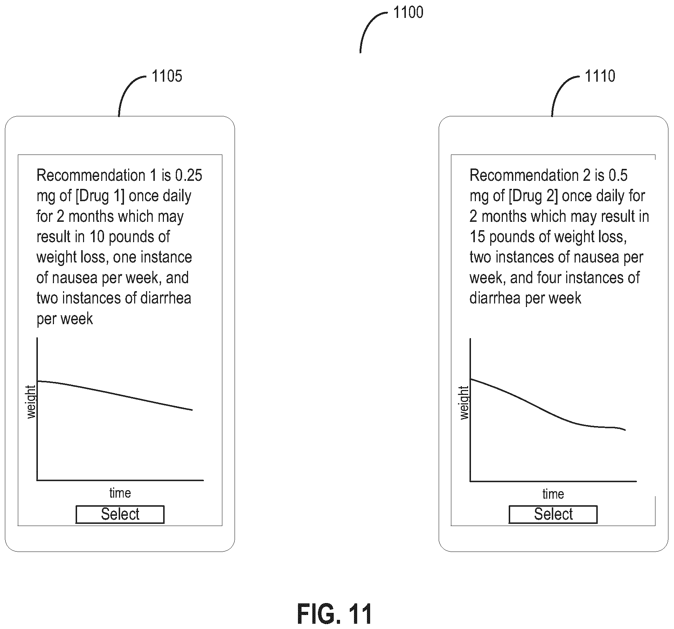

FIG. 11 depicts screenshots of a set 1100 of example user interfaces for selecting the medication, in accordance with an illustrative embodiment. The user interfaces in the set 1100 may be part of the application 125, and presented through the user interface 130. The user interface 1105 may be a prompt to the user to input values (e.g., one or more measurements) corresponding to the measurement fields displayed. The user can then input a numerical value, text string, or character string, among others, into the measurement fields. The user interfaces 1110 and 1115 both display weight loss medication recommendations, as well as predicted side effects and amount of weight loss over time. The user interfaces 1110 and 1115 also allow the user to select a medication based on the information provided by the user interfaces 1110 and 1115.

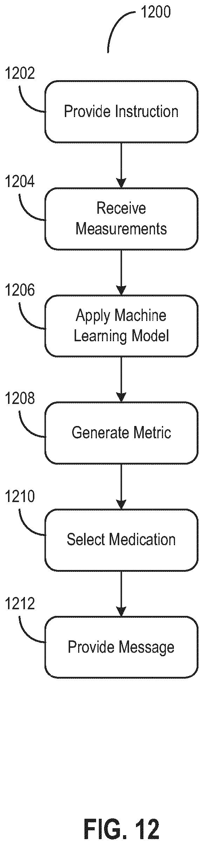

FIG. 12 depicts a flow diagram of a method 1200 for selecting a mediation for the user to address obesity in accordance with an illustrative embodiment. The method 1200 may be performed by any components of the system 100, such as the session management service 105, the user device 110, or the user 210, among others. Under the method 1200, one or more processors can provide an instruction (1202). The instruction can include one or more measurement fields for the user to provide input for. The one or more processors can receive measurements of a user (1204). The measurements can be input by the user and/or an instrumentation device worn by the user. The one or more processors can then apply the measurements to a machine learning model (1206). The machine learning model may be trained on a dataset including sample measurements, metrics, and medication IDs. The machine learning model can generate a metric (1208). The metric may be indicative of an expected outcome of a weight loss medication. The metric may be a set of metrics for each of a set of weight loss medications. The one or more processors may select a medication (1210). The one or more processors may select the medication based on a set of metrics for the set of weight loss medications. The one or more processors may provide a message (1212). The message may be based on the medication and the metric.

### B. Network and Computing Environment

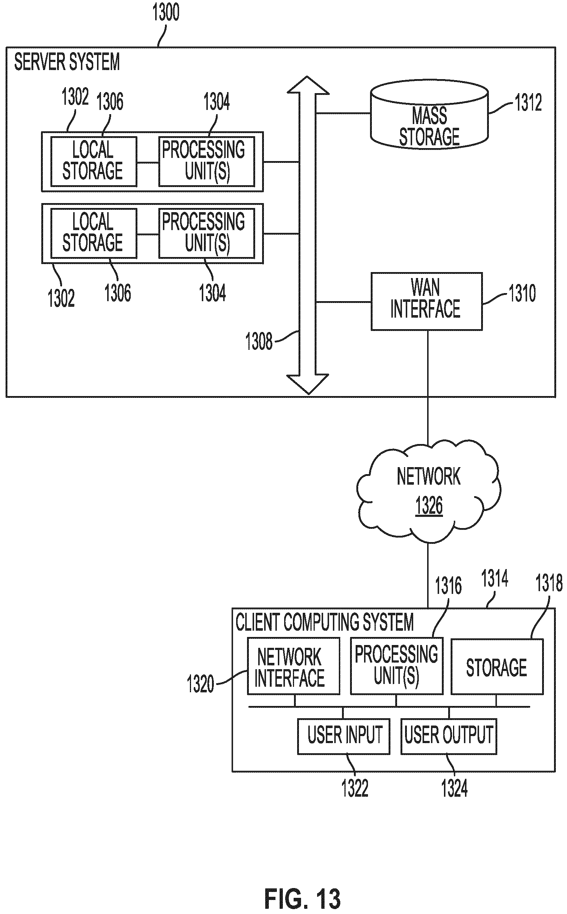

Various operations described herein can be implemented on computer systems. FIG. 13 shows a simplified block diagram of a representative server system 1300, client computing system 1314, and network 1326 usable to implement certain embodiments of the present disclosure. In various embodiments, server system 1300 or similar systems can implement services or servers described herein or portions thereof. Client computing system 1314 or similar systems can implement clients described herein. The system 100 described herein can be similar to the server system 1300. Server system 1300 can have a modular design that incorporates a number of modules 1302 (e.g., blades in a blade server embodiment); while two modules 1302 are shown, any number can be provided. Each module 1302 can include processing unit(s) 1304 and local storage 1306.

Processing unit(s) 1304 can include a single processor, which can have one or more cores, or multiple processors. In some embodiments, processing unit(s) 1304 can include a general-purpose primary processor as well as one or more special-purpose co-processors such as graphics processors, digital signal processors, or the like. In some embodiments, some or all processing unit(s) 1304 can be implemented using customized circuits, such as application specific integrated circuits (ASICs) or field programmable gate arrays (FPGAs). In some embodiments, such integrated circuits execute instructions that are stored on the circuit itself. In other embodiments, processing unit(s) 1304 can execute instructions stored in local storage 1306. Any type of processors in any combination can be included in processing unit(s) 1304.

Local storage 1306 can include volatile storage media (e.g., DRAM, SRAM, SDRAM, or the like) or non-volatile storage media (e.g., magnetic or optical disk, flash memory, or the like). Storage media incorporated in local storage 1306 can be fixed, removable, or upgradeable as desired. Local storage 1306 can be physically or logically divided into various subunits such as a system memory, a read-only memory (ROM), and a permanent storage device. The system memory can be a read-and-write memory device or a volatile read-and-write memory, such as dynamic random-access memory. The system memory can store some or all of the instructions and data that processing unit(s) 1304 need at runtime. The ROM can store static data and instructions that are needed by processing unit(s) 1304. The permanent storage device can be a non-volatile read-and-write memory device that can store instructions and data even when module 1302 is powered down. The term "storage medium" as used herein includes any medium in which data can be stored indefinitely (subject to overwriting, electrical disturbance, power loss, or the like) and does not include carrier waves and transitory electronic signals propagating wirelessly or over wired connections.

In some embodiments, local storage 1306 can store one or more software programs to be executed by processing unit(s) 1304, such as an operating system or programs implementing various server functions such as functions of the system 130 or any other system described herein, or any other server(s) associated with system 130 or any other system described herein.

"Software" refers generally to sequences of instructions that, when executed by processing unit(s) 1304, cause server system 1300 (or portions thereof) to perform various operations, thus defining one or more specific machine embodiments that execute and perform the operations of the software programs. The instructions can be stored as firmware residing in read-only memory or program code stored in non-volatile storage media that can be read into volatile working memory for execution by processing unit(s) 1304. Software can be implemented as a single program or a collection of separate programs or program modules that interact as desired. From local storage 1306 (or non-local storage described below), processing unit(s) 1304 can retrieve program instructions to execute and data to process in order to execute various operations described above.

In some server systems 1300, multiple modules 1302 can be interconnected via a bus or other interconnect 1308, forming a local area network that supports communication between modules 1302 and other components of server system 1300. Interconnect 1308 can be implemented using various technologies, including server racks, hubs, routers, etc.

A wide area network (WAN) interface 1310 can provide data communication capability between the local area network (e.g., through the interconnect 1308) and the network 1326, such as the Internet. Other technologies can be used to communicatively couple the server system with the network 1326, including wired (e.g., Ethernet, IEEE 802.3 standards) or wireless technologies (e.g., Wi-Fi, IEEE 802.11 standards).

In some embodiments, local storage 1306 is intended to provide working memory for processing unit(s) 1304, providing fast access to programs or data to be processed while reducing traffic on interconnect 1308. Storage for larger quantities of data can be provided on the local area network by one or more mass storage subsystems 1312 that can be connected to interconnect 1308. Mass storage subsystem 1312 can be based on magnetic, optical, semiconductor, or other data storage media. Direct attached storage, storage area networks, network-attached storage, and the like can be used. Any data stores or other collections of data described herein as being produced, consumed, or maintained by a service or server can be stored in mass storage subsystem 1312. In some embodiments, additional data storage resources may be accessible via WAN interface 1310 (potentially with increased latency).

Server system 1300 can operate in response to requests received via WAN interface 1310. For example, one of modules 1302 can implement a supervisory function and assign discrete tasks to other modules 1302 in response to received requests. Work allocation techniques can be used. As requests are processed, results can be returned to the requester via WAN interface 1310. Such operation can generally be automated. Further, in some embodiments, WAN interface 1310 can connect multiple server systems 1300 to each other, providing scalable systems capable of managing high volumes of activity. Other techniques for managing server systems and server farms (collections of server systems that cooperate) can be used, including dynamic resource allocation and reallocation.

Server system 1300 can interact with various user-owned or user-operated devices via a wide-area network such as the Internet. An example of a user-operated device is shown in FIG. 13 as client computing system 1314. Client computing system 1314 can be implemented, for example, as a consumer device such as a smartphone, other mobile phone, tablet computer, wearable computing device (e.g., smart watch, eyeglasses), desktop computer, laptop computer, and so on.

For example, client computing system 1314 can communicate via WAN interface 1310. Client computing system 1314 can include computer components such as processing unit(s) 1316, storage device 1318, network interface 1320, user input device 1322, and user output device 1324. Client computing system 1314 can be a computing device implemented in a variety of form factors, such as a desktop computer, laptop computer, tablet computer, smartphone, other mobile computing device, wearable computing device, or the like.

Processing unit 1316 and storage device 1318 can be similar to processing unit(s) 1304 and local storage 1306 described above. Suitable devices can be selected based on the demands to be placed on client computing system 1314. For example, client computing system 1314 can be implemented as a "thin" client with limited processing capability or as a high-powered computing device. Client computing system 1314 can be provisioned with program code executable by processing unit(s) 1316 to enable various interactions with server system 1300.

Network interface 1320 can provide a connection to the network 1326, such as a wide area network (e.g., the Internet) to which WAN interface 1310 of server system 1300 is also connected. In various embodiments, network interface 1320 can include a wired interface (e.g., Ethernet) or a wireless interface implementing various RF data communication standards such as Wi-Fi, Bluetooth, or cellular data network standards (e.g., 3G, 4G, LTE, etc.).

User input device 1322 can include any device (or devices) via which a user can provide signals to client computing system 1314; client computing system 1314 can interpret the signals as indicative of particular user requests or information. In various embodiments, user input device 1322 can include any or all of a keyboard, touch pad, touch screen, mouse or other pointing device, scroll wheel, click wheel, dial, button, switch, keypad, microphone, and so on.

User output device 1324 can include any device via which client computing system 1314 can provide information to a user. For example, user output device 1324 can include display-to-display images generated by or delivered to client computing system 1314. The display can incorporate various image generation technologies, e.g., a liquid crystal display (LCD), light-emitting diode (LED) display including organic light-emitting diodes (OLED), projection system, cathode ray tube (CRT), or the like, together with supporting electronics (e.g., digital-to-analog or analog-to-digital converters, signal processors, or the like). Some embodiments can include a device such as a touchscreen that function as both input and output device. In some embodiments, other user output devices 1324 can be provided in addition to or instead of a display. Examples include indicator lights, speakers, tactile "display" devices, printers, and so on.

Some embodiments include electronic components, such as microprocessors, storage, and memory that store computer program instructions in a computer readable storage medium. Many of the features described in this specification can be implemented as processes that are specified as a set of program instructions encoded on a computer readable storage medium. When these program instructions are executed by one or more processing units, they cause the processing unit(s) to perform various operations indicated in the program instructions. Examples of program instructions or computer code include machine code, such as is produced by a compiler, and files including higher-level code that are executed by a computer, an electronic component, or a microprocessor using an interpreter. Through suitable programming, processing unit(s) 1304 and 1316 can provide various functionality for server system 1300 and client computing system 1314, including any of the functionality described herein as being performed by a server or client, or other functionality.

It will be appreciated that server system 1300 and client computing system 1314 are illustrative and that variations and modifications are possible. Computer systems used in connection with embodiments of the present disclosure can have other capabilities not specifically described here. Further, while server system 1300 and client computing system 1314 are described with reference to particular blocks, it is to be understood that these blocks are defined for convenience of description and are not intended to imply a particular physical arrangement of component parts. For instance, different blocks can be but need not be located in the same facility, in the same server rack, or on the same motherboard. Further, the blocks need not correspond to physically distinct components. Blocks can be configured to perform various operations, e.g., by programming a processor or providing appropriate control circuitry, and various blocks might or might not be reconfigurable depending on how the initial configuration is obtained. Embodiments of the present disclosure can be realized in a variety of apparatus including electronic devices implemented using any combination of circuitry and software.

While the disclosure has been described with respect to specific embodiments, one skilled in the art will recognize that numerous modifications are possible. Embodiments of the disclosure can be realized using a variety of computer systems and communication technologies, including but not limited to specific examples described herein. Embodiments of the present disclosure can be realized using any combination of dedicated components or programmable processors or other programmable devices. The various processes described herein can be implemented on the same processor or different processors in any combination. Where components are described as being configured to perform certain operations, such configuration can be accomplished, e.g., by designing electronic circuits to perform the operation, by programming programmable electronic circuits (such as microprocessors) to perform the operation, or any combination thereof. Further, while the embodiments described above may make reference to specific hardware and software components, those skilled in the art will appreciate that different combinations of hardware or software components may also be used and that particular operations described as being implemented in hardware might also be implemented in software or vice versa.

Computer programs incorporating various features of the present disclosure may be encoded and stored on various computer readable storage media; suitable media include magnetic disk or tape, optical storage media such as compact disk (CD) or digital versatile disk (DVD), flash memory, and other non-transitory media. Computer readable media encoded with the program code may be packaged with a compatible electronic device, or the program code may be provided separately from electronic devices (e.g., via Internet download or as a separately packaged computer-readable storage medium).

Thus, although the disclosure has been described with respect to specific embodiments, it will be appreciated that the disclosure is intended to cover all modifications and equivalents within the scope of the following claims.

Example embodiments will be set out in the following numbered clauses, in which:
1. A method of selecting a weight loss medication for a user with an obesity condition, comprising:
   receiving, by one or more processors, one or more physiological measurements of the user;
   applying, by the one or more processors, the one or more physiological measurements to a machine learning model, wherein the machine learning model is trained using a plurality of examples, each example comprising one or more sample physiological measurements of a sample user and a corresponding sample weight loss medication administered to the sample user;
   generating, by the one or more processors, based on applying the one or more physiological measurements to the machine learning model, a metric indicating an expected outcome associated with a weight loss medication for the user; and
   providing, by the one or more processors, to a user device associated with the user, a message indicating at least one of a selection of a weight loss medication based on the metric indicating the expected outcome or the expected outcome associated with the weight loss medication.
2. The method of clause 1, further comprising selecting, by the one or more processors, the weight loss medication from a plurality of weight loss medications based on a plurality of expected outcomes associated with the plurality of weight loss medications.
3. The method of clause 1, wherein the one or more physiological measurements comprise at least one of: body-mass index, weight, blood pressure, heart rate, smoking status, glucose excretion, comprehensive metabolic panel, complete blood count, lipase levels, thyroid panel, magnesium level, HgA1c, fasting glucose, energy expenditure, physical activity, hormone levels, body weight, body fat percentage, a genetic marker, an evaluation of gut microbiome, or energy intake.
4. The method of clause 1, wherein the machine learning model comprises one or more corresponding weights to generate one or more values, the one or more corresponding weights comprising at least one of a binary weight or a continuous weight; and
   wherein generating the metric further comprises generating, by the one or more processors, the metric based on the one or more values.
5. The method of clause 1, wherein the weight loss medication is selected from a GLP-1 receptor agonist or a GIP receptor agonist.
6. The method of clause 5, wherein the GLP-1 receptor agonist is selected from one or more of semaglutide, liraglutide, exenatide, and dulaglutide, and wherein the GIP receptor agonist comprises tirzepatide.
7. The method of clause 1, wherein the expected outcome further comprises at least one administration parameter for the weight loss medication, the administration parameter comprising at least one of a dosage of the weight loss medication, a timing of administration of the weight loss medication, a frequency of administration, a route of administration, a dose escalation protocol, circumstances of administration, or any combination thereof.
8. The method of clause 1, wherein the expected outcome further comprises identifying at least one of a therapy discontinuation, side effects, or therapeutic efficacy.
9. The method of clause 8, wherein the side effects are selected from nausea, vomiting, diarrhea, early satiety, loss of appetite, anorexia, dizziness, increased heart rate, indigestion, headache, hypoglycemia, calculus of a kidney or ureter, pancreatitis, diabetic retinopathy, depression, suicidal ideation or attempts, pain in abdomen, acute kidney injury, muscle wasting and atrophy, constipation, or any combination thereof.
10. The method of clause 1, wherein generating the metric further comprises generating, based on applying the one or more physiological measurements to the machine learning model, a plurality of metrics for a plurality of expected outcome parameters.
11. The method of clause 8, wherein the expected outcome further comprises at least one expected outcome parameter, the expected outcome parameter comprising at least one of a timing of discontinuation, a cause of discontinuation, a probability of discontinuation, a discontinuation mitigation, or any combination thereof.
12. The method of clause 8, wherein the expected outcome further comprises at least one expected outcome parameter, the expected outcome parameter comprising at least one of timing of onset of the side effects, duration of the side effects, probability of the side effects, side effect mitigations, or any combination thereof.
13. The method of clause 8, wherein the expected outcome further comprises an expected outcome parameter, the expected outcome parameter comprising at least one of weight loss, fat loss, fasting blood glucose, cholesterol levels, hormone levels, duration of fat loss, risk of weight regain, a change in body mass index, or any combination thereof.
14. The method of clause 1, further comprising:
   generating, by the one or more processors, a simulation identifying a plurality of expected outcomes over a corresponding plurality of timepoints.
15. The method of clause 14, wherein the simulation comprises a representation of the plurality of expected outcomes over the corresponding plurality of timepoints, wherein the representation comprises at least one of a timeline, a graph, a video, an audio, or an avatar.
16. The method of clause 14, wherein the plurality of expected outcomes identified by the simulation comprise at least one expected outcome parameter, the expected outcome parameter comprising at least one of a timing of discontinuation, a cause of discontinuation, a probability of discontinuation, a discontinuation mitigation, or any combination thereof.
17. The method of clause 14, wherein the plurality of expected outcomes identified by the simulation comprise at least one expected outcome parameter, the expected outcome parameter comprising at least one of timing of onset of side effects, duration of side effects, probability of side effects, side effect mitigations, or any combination thereof.
18. The method of clause 14, wherein the plurality of expected outcomes identified by the simulation comprise an expected outcome parameter, the expected outcome parameter comprising at least one of weight loss, fat loss, fasting blood glucose, cholesterol levels, hormone levels, duration of fat loss, risk of weight regain, a change in body mass index, or any combination thereof.
19. The method of clause 1, wherein the user has a BMI greater than 25, a body fat percentage greater than 20%, Type I Diabetes, Type II Diabetes, or nonalcoholic steatohepatitis (NASH).
20. The method of clause 1, wherein the message is provided to the user device or a user's clinician.
21. The method of clause 1, wherein the one or more physiological measurements of the user are obtained by at least one of the user device or an instrumentation device on the user.
22. The method of clause 1, wherein the generating of the metric indicating the expected outcome comprises determining, by the one or more processors, the metric based on at least one of: (i) an average of the plurality of examples, (ii) a weighted combination of the plurality of examples, or (iii) a comparison with a dataset comprised of the plurality of examples.
23. The method of clause 1, wherein receiving the one or more physiological measurements further comprises receiving, by the one or more processors, over a time period, the one or more physiological measurements
   from at least one of the user device or an instrumentation device,
   wherein applying to the machine learning model further comprises applying, by the one or more processors, the one or more physiological measurements to the machine learning model, responsive to elapsing of the
      time period,
   wherein generating the metric further comprises generating, by the one or more processors, the metric indicating the expected outcome associated with the weight loss medication for a subsequent time period.
24. The method of clause 1, further comprising:
   receiving, by the one or more processors, one or more subsequent physiological measurements of the user;
   applying, by the one or more processors, responsive to receipt of the one or more subsequent physiological measurements, the one or more subsequent physiological measurements to the machine learning model;
   generating, by the one or more processors, based on applying the one or more subsequent physiological measurements to the machine learning model, a subsequent metric indicating a subsequent expected outcome associated with a subsequent weight loss medication for the user; and
   providing, by the one or more processors, to the user device within a defined time period relative to the receipt of the one or more subsequent physiological measurements, a subsequent message indicating at least one of:
      a) a selection of the weight loss medication or a subsequent weight loss medication based on the subsequent metric indicating the subsequent expected outcome, or
      b) the subsequent expected outcome associated with the subsequent weight loss medication.
25. The method of clause 24, wherein the defined time period ranges between 1 second to 1 hour.
26. A system, comprising:
   one or more processors configured to:
   receive one or more physiological measurements of the user;
   apply the one or more measurements to a machine learning model, wherein the machine learning model is trained using a plurality of examples, each example comprising one or more sample physiological measurements of a sample user and a corresponding sample weight loss medication administered to the sample user;
   generate, based on applying the one or more physiological measurements to the machine learning model, a metric indicating an expected outcome associated with a weight loss medication for the user; and
   provide to a user device associated with the user, a message indicating at least one of a selection of a weight loss medication based on the metric indicating the expected outcome or the expected outcome associated with the weight loss medication.
27. The system of clause 26, wherein the one or more processors are further configured to select the weight loss medication from a plurality of weight loss medications based on a plurality of expected outcomes associated with the plurality of weight loss medications.
28. The system of clause 26, wherein the one or more physiological measurements comprise at least one of: body-mass index, weight, blood pressure, heart rate, smoking status, glucose excretion, comprehensive metabolic panel, complete blood count, lipase levels, thyroid panel, magnesium level, HgA1c, fasting glucose, energy expenditure, physical activity, hormone levels, body weight, body fat percentage, a genetic marker, an evaluation of gut microbiome, or energy intake.
29. The system of clause 26, wherein the machine leaning model corresponding weights to generate one or more values, the one or more corresponding weights comprising at least one of a binary weight or a continuous weight;
   wherein to generate the metric, the one or more processors are further configured to generate the metric based on the one or more values.
30. The system of clause 26, wherein the weight loss medication is selected from a GLP-1 receptor agonist or a GIP receptor agonist.
31. The system of clause 30, wherein the GLP-1 receptor agonist is selected from one or more of semaglutide, liraglutide, exenatide, and dulaglutide, and wherein the GIP receptor agonist comprises tirzepatide.
32. The system of clause 26, wherein the expected outcome further comprises at least one administration parameter for the weight loss medication, the administration parameter comprising at least one of a dosage of the weight loss medication, a timing of administration of the weight loss medication, a frequency of administration, a route of administration, a dose escalation protocol, circumstances of administration, or any combination thereof.
33. The system of clause 26, wherein the expected outcome further comprises identifying at least one of a therapy discontinuation, side effects, or therapeutic efficacy.
34. The system of clause 33, wherein the side effects are selected from nausea, vomiting, diarrhea, early satiety, loss of appetite, anorexia, dizziness, increased heart rate, indigestion, headache, hypoglycemia, calculus of a kidney or ureter, pancreatitis, diabetic retinopathy, depression, suicidal ideation or attempts, pain in abdomen, acute kidney injury, muscle wasting and atrophy, constipation, or any combination thereof.
35. The system of clause 26, wherein to generate the metric, the one or more processors are further configured to generate, based on applying the one or more physiological measurements to the machine learning model, a plurality of metrics for a plurality of expected outcome parameters.
36. The system of clause 33, wherein the expected outcome further comprises at least one expected outcome parameter, the expected outcome parameter comprising at least one of a timing of discontinuation, a cause of discontinuation, a probability of discontinuation, a discontinuation mitigation, or any combination thereof.
37. The system of clause 33, wherein the expected outcome further comprises at least one expected outcome parameter, the expected outcome parameter comprising at least one of timing of onset of the side effects, duration of the side effects, probability of the side effects, side effect mitigations, or any combination thereof.
38. The system of clause 33, wherein the expected outcome further comprises an expected outcome parameter, the expected outcome parameter comprising at least one of weight loss, fat loss, fasting blood glucose, cholesterol levels, hormone levels, duration of fat loss, risk of weight regain, a change in body mass index, or any combination thereof.
39. The system of clause 26, wherein the one or more processors are further configured to generate a simulation identifying a plurality of expected outcomes over a corresponding plurality of timepoints.
40. The system of clause 39, wherein the simulation comprises a representation of the plurality of expected outcomes over the corresponding plurality of timepoints, wherein the representation comprises at least one of a timeline, a graph, a video, an audio, or an avatar.
41. The system of clause 39, wherein the plurality of expected outcomes identified by the simulation comprise at least one expected outcome parameter, the expected outcome parameter comprising at least one of a timing of discontinuation, a cause of discontinuation, a probability of discontinuation, a discontinuation mitigation, or any combination thereof.
42. The system of clause 39, wherein the plurality of expected outcomes identified by the simulation comprise at least one expected outcome parameter, the expected outcome parameter comprising at least one of timing of onset of side effects, duration of side effects, probability of side effects, side effect mitigations, or any combination thereof.
43. The system of clause 39, wherein the plurality of expected outcomes identified by the simulation comprise an expected outcome parameter, the expected outcome parameter comprising at least one of weight loss, fat loss, fasting blood glucose, cholesterol levels, hormone levels, duration of fat loss, risk of weight regain, a change in body mass index, or any combination thereof.
44. The system of clause 26, wherein the user has a BMI greater than 25, a body fat percentage greater than 20%, Type I Diabetes, Type II Diabetes, or nonalcoholic steatohepatitis (NASH).
45. The system of clause 26, wherein the message is provided to the user device or a user's clinician.
46. The system of clause 26, wherein the one or more physiological measurements of the user are obtained by at least one of the user device or an instrumentation device on the user.
47. The system of clause 26, wherein to generate the metric indicating the expected outcome, the one or more processors is further configured to determine comprises determining the metric based on at least one of: (i) an average of the plurality of examples, (ii) a weighted combination of the plurality of examples, or (iii) a comparison with a dataset comprised of the plurality of examples.
48. The system of clause 26, wherein to receive the one or more physiological measurements, the one or more processors are further configured to receive, over a time period, the one or more physiological measurements
   from at least one of the user device or an instrumentation device,
   wherein to apply the one or more physiological measurements to the machine learning model, the one or more processors are further configured to apply the one or
      more physiological measurements to the machine learning model, responsive to elapsing of the time period,
   wherein to generate the metric, the one or more processors are further configured to generate the metric indicating the expected outcome associated with the weight loss medication for a subsequent time period.
49. The system of clause 26, wherein the one or more processors are further configured to:
   receive one or more subsequent physiological measurements of the user;
   apply, responsive to receipt of the one or more subsequent physiological measurements, the one or more subsequent physiological measurements to the machine learning model;
   generate, based on applying the one or more subsequent physiological measurements to the machine learning model, a subsequent metric indicating a subsequent expected outcome associated with a subsequent weight loss medication for the user; and
   provide, to the user device within a defined time period relative to the receipt of the one or more subsequent physiological measurements, a subsequent message indicating at least one of:
      a) a selection of the weight loss medication or a subsequent weight loss medication based on the subsequent metric indicating the subsequent expected outcome, or
      b) the subsequent expected outcome associated with the subsequent weight loss medication.
50. The system of clause 49, wherein the defined time period ranges between 1 second to 1 hour.

## Claims

1. A method of selecting a weight loss medication for a user with an obesity condition, comprising:
receiving, by one or more processors, one or more physiological measurements of the user;
applying, by the one or more processors, the one or more physiological measurements to a machine learning model, wherein the machine learning model is trained using a plurality of examples, each example comprising one or more sample physiological measurements of a sample user and a corresponding sample weight loss medication administered to the sample user;
generating, by the one or more processors, based on applying the one or more physiological measurements to the machine learning model, a metric indicating an expected outcome associated with a weight loss medication for the user; and
providing, by the one or more processors, to a user device associated with the user, a message indicating at least one of a selection of a weight loss medication based on the metric indicating the expected outcome or the expected outcome associated with the weight loss medication.

2. The method of claim 1, further comprising selecting, by the one or more processors, the weight loss medication from a plurality of weight loss medications based on a plurality of expected outcomes associated with the plurality of weight loss medications.

3. The method of claim 1 or claim 2, wherein the one or more physiological measurements comprise at least one of: body-mass index, weight, blood pressure, heart rate, smoking status, glucose excretion, comprehensive metabolic panel, complete blood count, lipase levels, thyroid panel, magnesium level, HgA1c, fasting glucose, energy expenditure, physical activity, hormone levels, body weight, body fat percentage, a genetic marker, an evaluation of gut microbiome, or energy intake.

4. The method of any preceding claim, wherein the machine learning model comprises one or more corresponding weights to generate one or more values, the one or more corresponding weights comprising at least one of a binary weight or a continuous weight; and
wherein generating the metric further comprises generating, by the one or more processors, the metric based on the one or more values.

5. The method of any preceding claim, wherein the weight loss medication is selected from a GLP-1 receptor agonist or a GIP receptor agonist, and optionally
wherein the GLP-1 receptor agonist is selected from one or more of semaglutide, liraglutide, exenatide, and dulaglutide, and wherein the GIP receptor agonist comprises tirzepatide.

6. The method of any preceding claim, wherein the expected outcome further comprises at least one administration parameter for the weight loss medication, the administration parameter comprising at least one of a dosage of the weight loss medication, a timing of administration of the weight loss medication, a frequency of administration, a route of administration, a dose escalation protocol, circumstances of administration, or any combination thereof, or
wherein the expected outcome further comprises identifying at least one of a therapy discontinuation, side effects, or therapeutic efficacy, and optionally
wherein the side effects are selected from nausea, vomiting, diarrhea, early satiety, loss of appetite, anorexia, dizziness, increased heart rate, indigestion, headache, hypoglycemia, calculus of a kidney or ureter, pancreatitis, diabetic retinopathy, depression, suicidal ideation or attempts, pain in abdomen, acute kidney injury, muscle wasting and atrophy, constipation, or any combination thereof.

7. The method of any preceding claim, wherein generating the metric further comprises generating, based on applying the one or more physiological measurements to the machine learning model, a plurality of metrics for a plurality of expected outcome parameters.

8. The method of any of claims 6 or 7, wherein the expected outcome further comprises at least one expected outcome parameter, the expected outcome parameter comprising at least one of a timing of discontinuation, a cause of discontinuation, a probability of discontinuation, a discontinuation mitigation, or any combination thereof and/or
wherein the expected outcome further comprises at least one expected outcome parameter, the expected outcome parameter comprising at least one of timing of onset of the side effects, duration of the side effects, probability of the side effects, side effect mitigations, or any combination thereof, and/or
wherein the expected outcome further comprises an expected outcome parameter, the expected outcome parameter comprising at least one of weight loss, fat loss, fasting blood glucose, cholesterol levels, hormone levels, duration of fat loss, risk of weight regain, a change in body mass index, or any combination thereof.

9. The method of any preceding claim, further comprising:
generating, by the one or more processors, a simulation identifying a plurality of expected outcomes over a corresponding plurality of timepoints, and optionally
wherein the simulation comprises a representation of the plurality of expected outcomes over the corresponding plurality of timepoints, wherein the representation comprises at least one of a timeline, a graph, a video, an audio, or an avatar, and/or
wherein the plurality of expected outcomes identified by the simulation comprise at least one expected outcome parameter, the expected outcome parameter comprising at least one of a timing of discontinuation, a cause of discontinuation, a probability of discontinuation, a discontinuation mitigation, or any combination thereof, and/or
wherein the plurality of expected outcomes identified by the simulation comprise at least one expected outcome parameter, the expected outcome parameter comprising at least one of timing of onset of side effects, duration of side effects, probability of side effects, side effect mitigations, or any combination thereof, and/or
wherein the plurality of expected outcomes identified by the simulation comprise an expected outcome parameter, the expected outcome parameter comprising at least one of weight loss, fat loss, fasting blood glucose, cholesterol levels, hormone levels, duration of fat loss, risk of weight regain, a change in body mass index, or any combination thereof.

10. The method of any preceding claim, wherein the user has a BMI greater than 25, a body fat percentage greater than 20%, Type I Diabetes, Type II Diabetes, or nonalcoholic steatohepatitis (NASH).

11. The method of any preceding claim, wherein the message is provided to the user device or a user's clinician, and/or
wherein the one or more physiological measurements of the user are obtained by at least one of the user device or an instrumentation device on the user.

12. The method of any preceding claim, wherein the generating of the metric indicating the expected outcome comprises determining, by the one or more processors, the metric based on at least one of: (i) an average of the plurality of examples, (ii) a weighted combination of the plurality of examples, or (iii) a comparison with a dataset comprised of the plurality of examples.

13. The method of any preceding claim, wherein receiving the one or more physiological measurements further comprises receiving, by the one or more processors, over a time period, the one or more physiological measurements from at least one of the user device or an instrumentation device,
wherein applying to the machine learning model further comprises applying, by the one or more processors, the one or more physiological measurements to the machine learning model, responsive to elapsing of the time period,
wherein generating the metric further comprises generating, by the one or more processors, the metric indicating the expected outcome associated with the weight loss medication for a subsequent time period.

14. The method of any preceding claim, further comprising:
receiving, by the one or more processors, one or more subsequent physiological measurements of the user;
applying, by the one or more processors, responsive to receipt of the one or more subsequent physiological measurements, the one or more subsequent physiological measurements to the machine learning model;
generating, by the one or more processors, based on applying the one or more subsequent physiological measurements to the machine learning model, a subsequent metric indicating a subsequent expected outcome associated with a subsequent weight loss medication for the user; and
providing, by the one or more processors, to the user device within a defined time period relative to the receipt of the one or more subsequent physiological measurements, a subsequent message indicating at least one of:
a) a selection of the weight loss medication or a subsequent weight loss medication based on the subsequent metric indicating the subsequent expected outcome, or
b) the subsequent expected outcome associated with the subsequent weight loss medication, and optionally
wherein the defined time period ranges between 1 second to 1 hour.

15. A system, comprising:
one or more processors configured to perform the method of any one preceding claim.

## Amended claims

### Amended claims in accordance with Rule 137(2) EPC.

1. A method of selecting a weight loss medication (415) for a user (210) with an obesity condition, comprising:
receiving (1204), by one or more processors, one or more physiological measurements of the user;
applying (1206), by the one or more processors, the one or more physiological measurements to a machine learning model, wherein the machine learning model is trained using a plurality of examples, each example comprising one or more sample physiological measurements of a sample user and a corresponding sample weight loss medication administered to the sample user;
generating (1208), by the one or more processors, based on applying the one or more physiological measurements to the machine learning model, a metric indicating an expected outcome associated with a weight loss medication for the user;
generating, based on the metric, a customized recommendation comprising one or more administration parameters for the weight loss medication or comprising one or more preventative actions to be taken by the user; and
providing (1212), by the one or more processors, to a device (110) associated with the user or a clinician, the customized recommendation.

2. The method of claim 1, further comprising selecting (1210), by the one or more processors, the weight loss medication from a plurality of weight loss medications based on a plurality of expected outcomes associated with the plurality of weight loss medications.

3. The method of claim 1 or claim 2, wherein the one or more physiological measurements comprise at least one of: body-mass index, weight, blood pressure, heart rate, smoking status, glucose excretion, comprehensive metabolic panel, complete blood count, lipase levels, thyroid panel, magnesium level, HgA1c, fasting glucose, energy expenditure, physical activity, hormone levels, body weight, body fat percentage, a genetic marker, an evaluation of gut microbiome, or energy intake.

4. The method of any preceding claim, wherein the machine learning model comprises one or more corresponding weights to generate one or more values, the one or more corresponding weights comprising at least one of a binary weight or a continuous weight; and
wherein generating the metric further comprises generating, by the one or more processors, the metric based on the one or more values.

5. The method of any preceding claim, wherein the weight loss medication is selected from a GLP-1 receptor agonist or a GIP receptor agonist, and optionally
wherein the GLP-1 receptor agonist is selected from one or more of semaglutide, liraglutide, exenatide, and dulaglutide, and wherein the GIP receptor agonist comprises tirzepatide.

6. The method of any preceding claim, wherein the expected outcome further comprises at least one administration parameter for the weight loss medication, the administration parameter comprising at least one of a dosage of the weight loss medication, a timing of administration of the weight loss medication, a frequency of administration, a route of administration, a dose escalation protocol, circumstances of administration, or any combination thereof, or
wherein the expected outcome further comprises identifying at least one of a therapy discontinuation, side effects, or therapeutic efficacy, and optionally
wherein the side effects are selected from nausea, vomiting, diarrhea, early satiety, loss of appetite, anorexia, dizziness, increased heart rate, indigestion, headache, hypoglycemia, calculus of a kidney or ureter, pancreatitis, diabetic retinopathy, depression, suicidal ideation or attempts, pain in abdomen, acute kidney injury, muscle wasting and atrophy, constipation, or any combination thereof.

7. The method of any preceding claim, wherein generating the metric further comprises generating, based on applying the one or more physiological measurements to the machine learning model, a plurality of metrics for a plurality of expected outcome parameters.

8. The method of any of claims 6 or 7, wherein the expected outcome further comprises at least one expected outcome parameter, the expected outcome parameter comprising at least one of a timing of discontinuation, a cause of discontinuation, a probability of discontinuation, a discontinuation mitigation, or any combination thereof and/or
wherein the expected outcome further comprises at least one expected outcome parameter, the expected outcome parameter comprising at least one of timing of onset of the side effects, duration of the side effects, probability of the side effects, side effect mitigations, or any combination thereof, and/or
wherein the expected outcome further comprises an expected outcome parameter, the expected outcome parameter comprising at least one of weight loss, fat loss, fasting blood glucose, cholesterol levels, hormone levels, duration of fat loss, risk of weight regain, a change in body mass index, or any combination thereof.

9. The method of any preceding claim, further comprising:
generating, by the one or more processors, a simulation identifying a plurality of expected outcomes over a corresponding plurality of timepoints, and optionally
wherein the simulation comprises a representation of the plurality of expected outcomes over the corresponding plurality of timepoints, wherein the representation comprises at least one of a timeline, a graph, a video, an audio, or an avatar, and/or
wherein the plurality of expected outcomes identified by the simulation comprise at least one expected outcome parameter, the expected outcome parameter comprising at least one of a timing of discontinuation, a cause of discontinuation, a probability of discontinuation, a discontinuation mitigation, or any combination thereof, and/or
wherein the plurality of expected outcomes identified by the simulation comprise at least one expected outcome parameter, the expected outcome parameter comprising at least one of timing of onset of side effects, duration of side effects, probability of side effects, side effect mitigations, or any combination thereof, and/or
wherein the plurality of expected outcomes identified by the simulation comprise an expected outcome parameter, the expected outcome parameter comprising at least one of weight loss, fat loss, fasting blood glucose, cholesterol levels, hormone levels, duration of fat loss, risk of weight regain, a change in body mass index, or any combination thereof.

10. The method of any preceding claim, wherein the user has a BMI greater than 25, a body fat percentage greater than 20%, Type I Diabetes, Type II Diabetes, or nonalcoholic steatohepatitis (NASH).

11. The method of any preceding claim, wherein the customized recommendation is provided to the device, and/or
wherein the one or more physiological measurements of the user are obtained by at least one of the user device or an instrumentation device on the user.

12. The method of any preceding claim, wherein the generating of the metric indicating the expected outcome comprises determining, by the one or more processors, the metric based on at least one of: (i) an average of the plurality of examples, (ii) a weighted combination of the plurality of examples, or (iii) a comparison with a dataset comprised of the plurality of examples.

13. The method of any preceding claim, wherein receiving the one or more physiological measurements further comprises receiving, by the one or more processors, over a time period, the one or more physiological measurements from at least one of the device or an instrumentation device,
wherein applying to the machine learning model further comprises applying, by the one or more processors, the one or more physiological measurements to the machine learning model, responsive to elapsing of the time period,
wherein generating the metric further comprises generating, by the one or more processors, the metric indicating the expected outcome associated with the weight loss medication for a subsequent time period.

14. The method of any preceding claim, further comprising:
receiving, by the one or more processors, one or more subsequent physiological measurements of the user;
applying, by the one or more processors, responsive to receipt of the one or more subsequent physiological measurements, the one or more subsequent physiological measurements to the machine learning model;
generating, by the one or more processors, based on applying the one or more subsequent physiological measurements to the machine learning model, a subsequent metric indicating a subsequent expected outcome associated with a subsequent weight loss medication for the user;
generating, by the one or more processors, based on the subsequent metric, a subsequent customized recommendation comprising one or more subsequent administration parameters for the weight loss medication or comprising one or more subsequent preventative actions to be taken by the user; and
providing, by the one or more processors, to the device within a defined time period relative to the receipt of the one or more subsequent physiological measurements, the subsequent customized recommendation, and optionally wherein the defined time period ranges between 1 second to 1 hour.

15. A system (1300), comprising:
one or more processors (1304) configured to perform the method of any one preceding claim.
